# EUROPEAN PATENT APPLICATION

(11) **EP 1 398 373 A1**
(43) Date of publication of application: **17.03.2004**
(21) Application number: 02447169.0
(22) Date of filing: 06.09.2002
(51) Int. Cl.: C12N 9/20, C12P 23/00, C11D 3/386

(54) **Carotene specific lipases**

(71) Applicant: THE PROCTER & GAMBLE COMPANY, Cincinnati, Ohio 45202 (US)
(72) Inventor: Zorn, Holger, 30926 Seelze (DE); Takenberg, Meike, 30826 Garbsen (DE); Berger, Ralf G., 30455 Hannover (DE)
(74) Representative: Engisch, Gautier

(57) **Abstract**

Enzymes active in the hydrolysis of carotenoid esters have been obtained from *Pleurotus sapidus.* A particularly active enzyme has been identified having an isoelectric point of about 5.7 and a molecular weight of about 101kDA. A method of preparing carotenoid compounds from their respective esters comprises contacting the ester with the aforementioned enzyme. Carotene - comprising stains can be treated by the aforementioned enzyme, preferably followed by treatment with a conventional detergent or an enzyme active in cleaving carotenoids. A detergent comprising the aforementioned enzymes is also provided.

## Description

This invention relates to a carotene-specific lipase enzyme, detergent compositions comprising the lipase enzyme and methods for degrading carotenoid esters and methods for treating carotene-comprising stains.

Carotenoid substances are widely used as colorants and additives in food and animal feed as well as cosmetics. Carotenoids are found in many fruit and vegetables such as peppers, marigold, tomatoes etc. Most of the naturally occurring carotenoid substances are present as esters of fatty acids.

Free carotenoids have numerous advantages over their ester derivatives. The main advantage of the free carotenoids is their much improved bio-availability. This means that free carotenoids are much better resorbed by the digestive system and are therefore much healthier than their esterified counterparts. Apart from their colouring attributes, carotenoid rich plant extracts presently gain a great deal of attention in so called "functional foods". Radical scavenging properties are as well assigned to certain xanthophylls as preventive impacts on age-related macular degeneration (AMD) [Landrum JT, Bone RA Lutein, zeaxanthin, and the macular pigment. *Arch. Biochem. Biophys.* 2001, **385**, 28-40; Olmedilla B, Granado F, Blanco I, Vaquero M, Cajigal C Lutein in patients with cataracts and age related macular degenerations: a long term supplementation study. *J. Sci. Food Agric.* 2001, **81**, 904-909]. Several feeding studies proved that an optimal resorption is only warranted with free xanthophylls [Tyczkowski J, Hamilton PB Absorption, transport, and deposition in chickens of lutein diester, a carotenoid extracted from *marigold (Tagetes erecta)* petals. *Poult. Sci.* 1986, **65**, 1526-1531; Fletcher DL, Papa CM: The effect of saponification on the broiler coloring capability of marigold extracts. *Poult. Sci.* 1986, **65**, 1708-1714; Hencken H Chemical and physiological behaviour of feed carotenoids and their effects on pigmentation. *Poult. Sci.* 1992, **71**, 711-717]. As a consequence, there is an immense industrial demand for free carotenoids in the food and animal industry and in the manufacture of cosmetics.

Free carotenoids are used as colorants and/or additives for so-called functional foods. In particular, the naturally occurring oleoresins of *Tagetes erecta* L. (Marigold) and *Capsicum annuum* L. (red paprika), namely carotenoid esters in particular those of lutein and capsanthin, are commonly used as additives after converting them to their free form. According to estimations of leading manufacturers, up to 420 t of total xanthophylls from marigold are processed per year, and the annual demand for paprika oleoresin amounts to 1.400 t [Buckenhüskes HJ Aktuelle Anforderungen an Paprikapulver für die industrielle Verarbeitung. Z. Arzn. Gew.pfl. 1999, **4**, 111-118].

*Tagetes* is an ancient cultigen and medical plant originating from Latin America. The genus comprises several species, of which *Tagetes lucida* L. and *Tagetes erecta* L. are of predominant commercial relevance [Neher RT The ethnobotany of Tagetes. Econ. Bot. 1968, 22, 317-324]. The xanthophyll lutein is mainly responsible for the intense yellow coloration of the crown petals and, to a minor extent, in some species also zeaxanthin (approx. 4%). More than 95% of the lutein occurs in the form of esters with fatty acids of varying chain length and degree of unsaturation [Breithaupt DE, Wirt U, Bamedi A Differentiation between lutein monoester regioisomers and detection of lutein diesters from marigold flowers (*Tagetes erecta* L.) and several fruits by liquid chromatography-mass spectrometry. *J. Agric.* Food Chem. 2002, **50**, 66 - 70; Gregory GK, Chen T-S, Philip T Quantitative analysis of lutein esters in marigold flowers (*Tagetes erecta)* by high performance liquid chromatography. *J. Food Sci.* 1986, **51**, 1093-1094; Rivas JDL Reversed-phase high-performance liquid chromatographic separation of lutein and lutein fatty acid esters from marigold flower petal powder. *J. Chromatogr.* 1989, **464**, 442-227].

The carotenoid spectrum of ripe paprika fruits is dominated by capsanthin. 80% of these carotenoids are in the form of fatty acid diesters within the mesocarp. Minor paprika carotenoids are □-carotene and the likewise preponderantly mono- and diesterified □-cryptoxanthin, zeaxanthin, capsorubin, and violaxanthin [Breithaupt DE, Schwack W Determination of free and bound carotenoids in paprika (*Capsicum annuum* L.) by LC/MS. *Eur. Food Res. Technol.* 2000, **211**, 52-55; Mìnguez-Mosquera Ml, Hornero-Méndez D Changes in carotenoid esterification during the fruit ripening of *Capsicum annuum cv. Bola. J. Agric. Food Chem.* 1994, **42**, 640-644; Biacs PA, Daood HG, Pavisa A, Hajdu F Studies on the carotenoid pigments of paprika (*Capsicum annuum* L. var Sz-20). *J. Agric. Food Chem.* 1994, **37**, 350-353].

In order to obtain free carotenoids from their ester precursors, the conventional method of chemical saponification is usually used. In general, temperatures of up to 80°C and concentrated sodium hydroxide solution are applied to obtain complete ester hydrolysis. However, the technical saponification of carotenoid esters in this way is associated with serious disadvantages. The use of concentrated alkali and elevated temperatures causes product impairment. ln addition, the chemical hydroxide saponification methods involve high costs for energy. There has therefore been a long-standing desire to find an alternative method of converting carotenoid esters to free carotenoids.

Enzyme catalysis has been considered as a possible route for hydrolysing carotenoid esters. However, the need for high priced bile salts and slow conversion rates has so far hampered the establishment of an efficient bioprocess to hydrolyse carotenoid esters using commercially available enzymes.

To date, there are only very few enzymes known which catalyse carotenoid ester hydrolysis at all. lnvestigations on lutein, zeaxanthin, and β-cryptoxanthin esters in human milk and serum have been published by Khachik *et al* [Khachik F, Spangler CJ, Smith Jr JC ldentification, quantification, and relative concentrations of carotenoids and their metabolites in human milk and serum. *J. Agric. Food Chem.* 1997, **69**, 1873-1881] and Liu *et al* [Liu Y, Xu MJ, Canfield LM Enzymatic hydrolysis, extraction, and quantitation of retinol and major carotenoids in mature human milk. *J. Nutr. Biochem.* 1998, **9**, 178-183]. Capsanthin esters were partly hydrolysed by a lipase from *Candida rugosa* [Breithaupt DE Enzymatic hydrolysis of carotenoid fatty acid esters of red pepper (*Capsicum annuum* L.) by a lipase from *Candida rugosa. Z. Naturforsch.* 2000, **55c**, 971-976]. None of these enzymes catalyse ester hydrolysis of carotenoid esters very efficiently.

Due to their comparatively broad substrate acceptance, lipases from Candida species play an important role in biotechnological applications [Benjamin S, Pandey A Candida rugosa lipase: molecular biology and versatility in biotechnology. *Yeast* 1998, **14**, 1069-1087]. Crystal structures are available for several representatives and their reactivity and the function of the lid region is well understood [Grochulski P, LiY, Schrag JD, Bouthillier F, Smith P, Harrison D, Rubin B, Cygler M Insights into interfacial activation from an open structure of *Candida rugosa* lipase. *J. Biol. Chem.* 1993, **268**, 12843-12847; Uppenberg J, Öhrner N, Norin M Crystallographic and molecular-modeling studies of lipase B from *Candida antarctica* reveal a stereospecifity pocket for secondary alcohols. *Biochem* 1995, **34,** 16838-16851]. However, none of these enzymes yield release rates beyond 44% for the lutein and 69% for the capsanthin esters, respectively.

Another disadvantage of the known enzymes is that bile salts are essential for their activity.

As noted, carotenoid compounds possess intense coloration. Their use in food, cosmetics and other products leads to problems arising from this coloration. Carotene-based stains derived from natural sources are often difficult to remove from fabrics, clothing and other material, in particular porous material. Conventional detergents based on chemicals often fails to completely remove such stains.

Accordingly, there is a need for an improved enzyme capable of catalysing enzymatic ester hydrolysis of carotenoid esters. Such an enzyme would be of particular use in the preparation of carotenoids from their esters and in the treatment of stains.

Unexpectedly, a novel enzyme was found to have excellent properties with regard to converting carotenoid esters into free carotenoids. The novel lipase found possesses an isoelectric point of about 5.7 and a molecular weight of about 101 kDa. ln particular, it was found that the basidiomycete *P. sapidus* produces a lipase enzyme which is capable of very efficiently and gently hydrolysing carotenoid esters to release the free carotenoids to high levels of conversion.

Though various basidiomycetes have been valued tools in the cellulose processing industry for a long time and enormous efforts have been made to characterize their lignolytic enzyme systems, only little has become known about their lipolytic properties [Rajarathnam S, Shashirekha MnjU, Bano Z Biodegradative and Biosynthetic Capacities of Mushrooms: Present and Future Strategies. *Crit. Rev. Biotechnol.* 1998, **18** (2&3), 91-236]. There are no known basidiomycete lipases which have carotenoid substrate specificity.

*P. sapidus,* a close relative to the oyster mushroom (*P. ostreatus),* is a highly valued edible mushroom. As a result of the extensive experience with various *Pleurotus* species as foodstuffs, toxicological risks are negligible. The lipolytic activity is secreted into the culture supernatant, which facilitates the handling of the bioprocess as well as a future enzyme enrichment and characterisation.

*P. sapidus* is a commercially available organism (DSMZ 8266, Deutsche Sammlung für Mikrooganismen und Zellkulturen GmbH, Braunschweig, Germany). *P. sapidus* is easily cultivated in submerged cultures with minimum demands for medium supplements and thus represents an ideal tool for biotechnological applications. The cultures environmentally compatible mild conditions with moderate temperatures, typically in the range of from 10 to 35°C, preferably from 15 to 30°C, with about 25°C being optimum. The culture may be affected in an environment having a pH of from about 6 to 8, with a pH of about 7 being preferred. *P. sapidus* is preferably grown in conditions of low light, darkness being preferred.

The lipase of the present invention is found in the mycelium free supernatant of submerged cultures of *P. sapidus.* The lipase according to the present invention has been biochemically characterised and found to have an isoelectric point of about 5.7 and a molecular weight of about 101 kDa. These characteristics were determined using IEF and SEC analyses as described in Example 3.

The lipase of the present invention cleaves carotenoid esters, many almost completely, and is active in the absence of bile salts. This is a great advantage since the addition of bile salts often damages the desired end products.

The lipase according to the present invention has a broad substrate range among the carotenoid esters, in particular xanthophyllesters and including mono - and diesterified □-cryptoxanthin, capsorubin, mutatoxanthin, luteoxanthin violaxanthin. capsanthin ester, cis-capsanthin ester, *all-trans*-capsanthin ester, zeaxanthin ester, and lutein ester.

The conversion efficiency achieved with the novel enzyme is much higher than with known enzymes. Conversion ranges from about 69% to almost complete conversion, preferably at least 80%, more preferably at least 90%. The conversion efficiency for lutein esters is particularly high.

The enzyme is preferably produced from a culture of *P. sapidus.* Preferably, a submerged culture of *P. sapidus* is grown in standard nutrient medium using techniques known in the art, as illustrated in Example 3. The mycelia are separated using known techniques, such as centrifugation. The supernatant can be used directly to convert carotenoid esters to free carotenoids, or it can be further purified using techniques known in the art, such as ultra filtration or precipitation.

Another aspect of the present invention provides a method for producing free carotenoids from their ester derivatives comprising contacting a carotenoid ester with the lipase enzyme of the invention. The method preferably further comprises isolating free carotenoids. Preferably, the method is carried out in the absence of bile salts.

In a preferred embodiment, a cell-free culture supernatant of *P. sapidus*, produced as described above and containing the lipase of the invention, is employed. In an alternative preferred embodiment, a purified lipase enzyme is used.

The carotenoid esters that can be converted to their free forms using the method of the present invention, are preferably derived from a plant source, in particular carotene containing vegetables and fruit such as *Capsicum annuum* L. (peppers) and/or *Tagetes erecta* L (marigold). These plants contain many carotenoids, the majority of which are present as esters, mainly with fatty acids. Preferably, xanthophyll esters are used to obtain free carotenoids, in particular capsanthin ester, *cis*-capsanthin ester, *all-trans*-capsanthin ester, zeaxanthin ester, and lutein ester.

The lipase of the present invention catalyses the hydrolysis of di-esters to mono-esters and of mono-esters to free carotenoids. The free carotenoids obtainable with the method according to the present invention are very useful as fragrance- and/or flavour precursors for the use in perfumes and/or foods and animal feed. Due to their colour, they are also useful as colorants in many foods. Free carotenoids, in particular xanthophylls are preferred additives for cattle feed.

By using the lipase of the present invention, it is also possible to improve natural products such as natural food and cosmetic products. Therefore, another aspect of the invention is the treatment of natural food products with the lipase of the present invention. Examples of such natural food products are extract of fruit, vegetables, foliage, herbs and similar natural products which contain esterified carotenoids. These may be in further processed form such as in the form of extracts, juices, purees, pulps or the like, such as used in baby food and other processed food, or they may be dried such as dried leaves, petals, fruit, e.g. for use in herbal infusions. Cattle feed comprising natural fruit products, herbs and vegetables is also a preferred application for the use of the lipase of the invention. The treatment of such natural products with the lipase will lead to the release of free carotenoids from the carotenoid esters naturally occurring in those products. This will, in turn, improve the food products with regard to bio-availability and will make them healthier. ln addition, the lipase treatment as mentioned above can be combined with further enzyme treatment, e.g. with an enzyme which further cleaves the free carotenoids to improve the flavour and/or fragrance of the food products.

Another aspect of the present invention relates to a method for treating carotene-comprising stains. The lipase according to the present invention can be used in a method to treat carotene stains by releasing the free carotenoids from their ester derivatives. The free carotenoids are then more easily removed from the stained object, for example using known detergent compositions.

According to the method of the invention, the stained material for example a fabric, is contacted with a lipase according to the invention. Preferably, the stain is subsequently treated with a further detergent and rinsed to at least partially remove the stain. The further detergent can be any suitable detergent known in the art and preferably comprises one or more surfactants, dispersants, balance carriers and adjunct ingredients. In a preferred embodiment of the method, the stain is additionally contacted with a carotene-cleaving chemical and/or a carotene-cleaving enzyme, such as a peroxidase.

Another aspect of the present invention is a detergent composition, comprising a lipase of the present invention. The detergent composition may be formulated in manners known in the art and may contain one or more surfactants, dispersants, balance carriers or adjunct ingredients. In a preferred embodiment, the detergent composition additionally comprises a carotene-cleaving chemical and/or a carotene-cleaving enzyme, such as a peroxidase.

The detergent compositions in which the enzyme of the invention may be incorporated comprise a variety of components, and levels of incorporation thereof will depend on the physical form of the composition, and the nature of the cleaning operation for which it is to be used. lndeed, the detergent compositions herein include laundry detergents as well as hard surface cleaners, hand dishwashing or automatic dishwashing detergents. The detergent compositions herein can be liquid, paste, gels, bars, tablets, spray, foam, powder or granular. Granular compositions can also be in "compact" form and the liquid compositions can also be in a "concentrated" form. Tablet compositions can be in single phase or multiple phase form.

When formulated as compositions for use in manual dishwashing methods the compositions herein typically contain a surfactant and preferably other detergent compounds selected from organic polymeric compounds, suds enhancing agents, group II metal ions, solvents, hydrotropes and additional enzymes.

When formulated as compositions suitable for use in a laundry machine washing method, the compositions herein typically contain both a surfactant and a builder compound and additionally one or more detergent components preferably selected from organic polymeric compounds, bleaching agents, additional enzymes, suds suppressors, dispersants, lime-soap dispersants, soil suspension and anti-redeposition agents and corrosion inhibitors. Laundry compositions can also contain softening agents, as additional detergent components.

When formulated as compositions suitable for use in a machine dish wash method, the compositions herein typically contain a low foaming nonionic surfactant, a builder system, and one or more components preferably selected from organic polymeric compounds, bleaching agents, additional enzymes, suds suppressors, dispersants, lime-soap dispersants, soil suspension and anti-redeposition agents and corrosion inhibitors.

The compositions herein can also be used as detergent additive products in solid or liquid form. Such additive products are intended to supplement or boost the performance of conventional detergent compositions and can be added at any stage of the cleaning process.

lf needed the density of the laundry detergent compositions herein ranges from 400 to 1200 g/litre, preferably 500 to 950 g/litre of composition measured at 20°C. The "compact" form of the compositions herein is best reflected by density and, in terms of composition, by the amount of inorganic filler salt; inorganic filler salts are conventional ingredients of detergent compositions in powder form; in conventional detergent compositions, the filler salts are present in substantial amounts, typically 17-35% by weight of the total composition. In the compact compositions, the filler salt is present in amounts not exceeding 15% of the total composition, preferably not exceeding 10%, most preferably not exceeding 5% by weight of the composition. The inorganic filler salts, such as meant in the present compositions are selected from the alkali and alkaline-earth-metal salts of sulphates and chlorides. A preferred filler salt is sodium sulphate.

Liquid detergent compositions according to the present invention can also be in a "concentrated form", in such case, the liquid detergent compositions according the present invention will contain a lower amount of water, compared to conventional liquid detergents. Typically the water content of the concentrated liquid detergent is preferably less than 40%, more preferably less than 30%, most preferably less than 20% by weight of the detergent composition.

The compounds which are generally suitable for use in detergents are described as follows.

Detergent compositions typically comprise a surfactant system wherein the surfactant can be selected from cationic, nonionic and/or conventional anionic and/or mixtures thereof. Also suitable are ampholytic and/or zwitterionic and/or semi-polar surfactants. The surfactant system is typically present at a level of from 0.1 % to 60% by weight. More preferred levels of incorporation are 1 % to 35% by weight, most preferably from 1 % to 30% by weight of the detergent compositions.

The detergent composition may comprise one or more bleaching agents. Bleaching agents that can be used encompasses peroxygen bleaches and halogen bleaching agents. Examples of peroxygen bleaches are inorganic perhydrate bleaches, typically percarbonates and perborates, use alone or in combination with bleach activators such as TAED. Examples of hypohalite bleaching agents, for example, include trichloro-isocyanuric acid and the sodium and potassium dichloroisocyanurates and N-chloro and N-bromo alkane sulphonamides. Such materials are normally added at 0.5-10% by weight of the finished product, preferably 1-5% by weight.

Bleaching agents other than oxygen bleaching agents are also known in the art and can be utilized herein. One type of non-oxygen bleaching agent of particular interest includes photoactivated bleaching agents such as the sulfonated zinc and/or aluminium phthalocyanines. These materials can be deposited upon the substrate during the washing process. Upon irradiation with light, in the presence of oxygen, such as by hanging clothes out to dry in the daylight, the sulfonated zinc phthalocyanine is activated and, consequently, the substrate is bleached. Preferred zinc phthalocyanine and a photoactivated bleaching process are described in U.S. Patent 4,033,718. Typically, detergent compositions will contain about 0.025% to about 1.25%, by weight, of sulfonated zinc phthalocyanine.

The detergent compositions herein can further comprise a builder. Any conventional builder system is suitable for use herein including aluminosilicate materials, silicates, polycarboxylates, alkyl- or alkenyl-succinic acid and fatty acids, materials such as ethylenediamine tetraacetate, diethylene triamine pentamethyleneacetate, metal ion sequestrants such as aminopolyphosphonates, particularly ethylenediamine tetramethylene phosphonic acid and diethylene triamine pentamethylenephosphonic acid. Phosphate builders can also be used herein. lnorganic aluminosilicates, commonly known as zeolites are also suitable for use herein.

Detergency builder salts are normally included in amounts of from 5% to 80% by weight of the composition preferably from 10% to 70% and most usually from 30% to 60% by weight.

The detergent compositions can, in addition to the enzyme herein, further comprise one or more enzymes which provide cleaning performance, fabric care and/or sanitisation benefits. Said enzymes include enzymes selected from cellulases, hemicellulases, peroxidases, proteases, gluco-amylases, amylases, xylanases, lipases, phospholipases, esterases, cutinases, other pectinases, keratanases, reductases, oxidases, phenoloxidases, lipoxygenases, ligninases, pullulanases, tannases, pentosanases, malanases, β-glucanases, arabinosidases, hyaluronidase, chondroitinase, laccase, pectin lyase, pectate lyase or mixtures thereof. A preferred combination is a detergent composition having a cocktail of conventional applicable enzymes like protease, amylase, lipase, cutinase and/or cellulase in conjunction with one or more plant cell wall degrading enzymes.

Enzymes are normally incorporated in the detergent composition at a total level of from 0.0001 % to 2% of pure enzyme by weight of the detergent composition. The enzymes can be added as separate single ingredients (prills, granulates, stabilized liquids, etc. containing one enzyme ) or as mixtures of two or more enzymes (e.g. cogranulates ).

Technologies which provide a type of colour care benefit can also be included. Examples of these technologies are metallo catalysts for colour maintenance. Such metallo catalysts are described in EP-B-0 596 184. Dye fixing agents, polyolefin dispersion for anti-wrinkles and improved water absorbancy, perfume and amino-functional polymer, disclosed in EP-A-0 931 133, for colour care treatment and perfume substantivity are further examples of colour care / fabric care technologies.

Fabric softening agents can also be incorporated into detergent compositions in accordance with the present invention. These agents may be inorganic or organic in type. lnorganic softening agents are exemplified by the smectite clays disclosed in GB-A-1 400 898 and in USP 5,019,292. Organic fabric softening agents include the water insoluble tertiary amines as disclosed in GB-A1 514 276 and EP-B0 011 340 and their combination with mono C12-C14 quaternary ammonium salts are disclosed in EP-B-0 026 527 and EP-B-0 026 528 and di-long-chain amides as disclosed in EP-B-0 242 919. Other useful organic ingredients of fabric softening systems include high molecular weight polyethylene oxide materials as disclosed in EP-A-0 299 575 and 0 313 146.

Levels of smectite clay are normally in the range from 2% to 20%, more preferably from 5% to 15% by weight, with the material being added as a dry mixed component to the remainder of the formulation. Organic fabric softening agents such as the water-insoluble tertiary amines or dilong chain amide materials are incorporated at levels of from 0.5% to 5% by weight, normally from 1 % to 3% by weight whilst the high molecular weight polyethylene oxide materials and the water soluble cationic materials are added at levels of from 0.1 % to 2%, normally from 0.15% to 1.5% by weight. These materials are normally added to the spray dried portion of the composition, although in some instances it may be more convenient to add them as a dry mixed particulate, or spray them as molten liquid on to other solid components of the composition.

The detergent compositions herein may also optionally contain one or more iron and/or manganese chelating agents. Such chelating agents can be selected from the group consisting of amino carboxylates, amino phosphonates, polyfunctionally-substituted aromatic chelating agents and mixtures therein, all as hereinafter defined. Without intending to be bound by theory, it is believed that the benefit of these materials is due in part to their exceptional ability to remove iron and manganese ions from washing solutions by formation of soluble chelates.

lf utilized, these chelating agents will generally comprise from about 0.1 % to about 15% by weight of the detergent compositions herein. More preferably, if utilized, the chelating agents will comprise from about 0.1 % to about 3.0% by weight of such compositions.

Other components used in detergent compositions may be employed, such as soil-suspending agents, soil-release agents, optical brighteners, abrasives, bactericides, tarnish inhibitors, suds suppressors, dye transfer inhibitors, colouring agents, and/or encapsulated or non-encapsulated perfumes, dispersants.

The compositions of the invention may be used in essentially any washing or cleaning methods, including soaking methods, pre-treatment methods and methods with rinsing steps for which a separate rinse aid composition may be added.

The process described herein comprises contacting fabrics, dishware or any other hard surface with a cleaning solution in the usual manner and exemplified hereunder. A conventional laundry method comprises treating soiled fabric with an aqueous liquid having dissolved or dispensed therein an effective amount of the laundry detergent and/or fabric care composition. A preferred machine dishwashing method comprises treating soiled articles with an aqueous liquid having dissolved or dispensed therein an effective amount of the machine dishwashing or rinsing composition. A conventional effective amount of the machine dishwashing composition means from 8-60 g of product dissolved or dispersed in a wash volume from 3-10 litres. According to a manual dishwashing method, soiled dishes are contacted with an effective amount of the dishwashing composition, typically from 0.5-20g (per 25 dishes being treated). Preferred manual dishwashing methods include the application of a concentrated solution to the surfaces of the dishes or the soaking in large volume of dilute solution of the detergent composition. A conventional hard surface method comprises treating soiled hard items/surfaces with e.g. a sponge, brush, clothe, etc. with an aqueous liquid having dissolved or dispensed therein an effective amount of the hard surface cleaner and/or with such composition undiluted. It also encompasses the soaking of a hard item in a concentrated solution or in a large volume of dilute solution of the detergent composition.

The process of the invention is conveniently carried out in the course of the cleaning process. The method of cleaning is preferably carried out at 5°C to 95°C, especially between 10°C and 60°C. The pH of the treatment solution is preferably from 7 to 12.

The present invention will now be described by way of illustration in the following specific examples, having reference to the accompanying figures, in which:
- **Figure 1**: depicts a Coomassie stained gel (A) and an activity stained gel (B) of the lipase of the present invention.
- **Figures 2 and 3**: show the result of the determination of the iso-electric point.
- **Figure 4**: depicts a chromatogram of samples obtained by chemical saponification of *Tagetes erecta* derived lutein diesters using potassium hydroxide (**a**) and *Pleurotus sapidus* culture supernatant (**b**). Chromatogram (**c**) represents the native lutein diester pattern of marigold oleoresin.
- **Figure 5**: depicts a chromatogram (DAD, 450 nm) of samples obtained by chemical saponification of *Capsicum annuum* derived xanthophyll esters (mainly capsanthin diesters) using potassium hydroxide (**a**) and *Pleurotus sapidus* culture supernatant (**b**). Chromatogram (**c**) represents the native carotenoid diester pattern of paprika oleoresin.

### Examples

### Example 1

### Biochemical characterisation of the lipase from Pleurotus sapidus

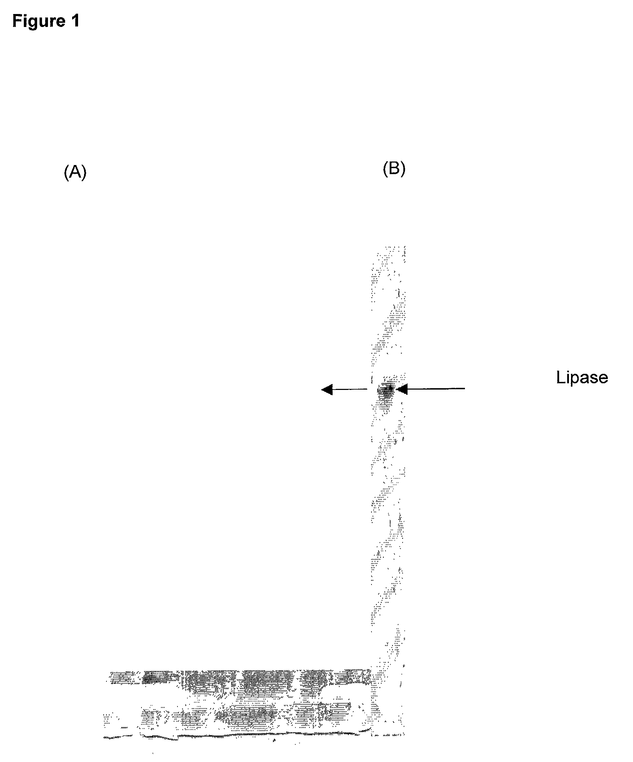

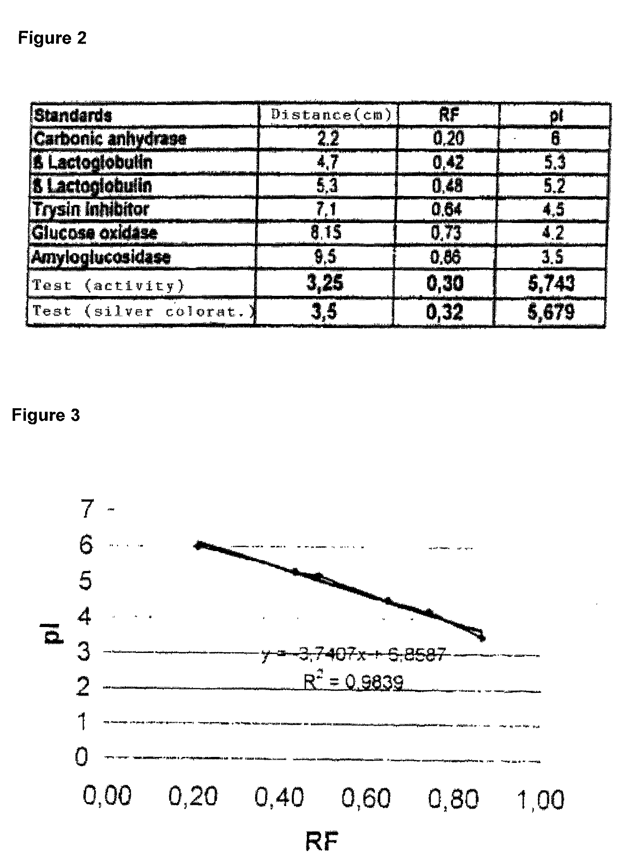

The biochemical characterisation of the lipase from *P. sapidus* comprised the determination of the iso-electric point by IEF analysis by activity staining (Figures 1 to 3) and a molecular weight analysis by means of size exclusion chromatography
Determination of the molecular weight by means of size exclusion chromatography (SEC): The mycelium free culture supernatant of *P. sapidus* was concentrated by means of ultra-filtration (Centricon plus 80 filter (Millipore, Bedford, US); 30 kDa exclusion limit; 5 °C, 3000 × g, 20 min). 200 µL of the retentate were loaded on a Superdex 200 HR SEC column (Amersham Biotech, Uppsala, Sweden), and 20 mM Tris-HCl-buffer + 1 mM CaCl₂ (pH 6.8) served as eluent (flow rate 0.5 mL min⁻¹). Fractions of 1 mL were cut and lipase activity was determined in each fraction.
Activity test: 60 mL of solution B (0.008 g Tris-HCl + 0.025 g NaCl/100 mL H₂O) and 1.2 g Agar Agar were heated to 100 °C for 1 min. After cooling to ∼65 °C, 60 mL of solution A (1 g Tween™ 80 + 0.02 g CaCl₂/100 mL H₂O; pH adjusted to 6.8 with 0.1 N NaOH) were added. The mixture was poured into Petri dishes and after curing of the agar plates small holes (5 mm diameter) were stamped out using a cork borer. The holes were filled each with 30 µL of a SEC fraction and after incubation at 35 °C for 10 h the plates were covered with 0.1 N NaOH. Forming of a whitish precipitate around a hole indicated active fractions.
For molecular weight analysis the SEC was calibrated using the reference proteins catalase (232 kDa), thyroglobulin (669 kDa), ferritin (440 kDa), aldolase (158 kDa), and ribonuclease (13.7 kDa). The void volume of the column was determined using Blue dextran 2000.
Sample preparation for IEF analysis: The mycelium free culture supernatant of *P. sapidus* was concentrated by means of ultra-filtration (see above) and the ultra-filtration retentate was fractionated by SEC as described above. SEC fraction 16, which showed highest activity in the precipitation test (see above), was concentrated and desalted by ultra-filtration (Ultrafree (Millipore, Bedford, USA); 30 kDa exclusion limit; 5 °C, 3000 × g, 20 min). 3, 6, and 12 µL of the thus prepared sample were loaded on a Servalyt Precotes pH 3 - 6 gel (Serva, Heidelberg, Germany)

The iso-electric point was determined to be about 5.7. The lipase was determined to have a molecular weight of about 101 kDa

### Example 2

### Comparative Example: Activity of known enzymes

To study the specificity of commercial available lipases on carotenoid esters, an enzymatic assay described by Breithaupt [see above] was applied:

Light petroleum (boiling fraction 40-60 °C), methanol, ethyl acetate, tert-butyl methyl ether, and diethyl ether were obtained from BASF (Ludwigshafen, Germany), potassium hydroxide, butylated hydroxytoluene, and bile salts (cholic acid-deoxycholic acid sodium salt mixture) were obtained from Fluka (Neu-Ulm, Germany). All solvents were distilled before use.

High-purity water was prepared with a Milli-Q 185 plus water purification system (Millipore, Eschborn, Germany). Capsanthin and lutein were obtained from Hoffmann-La Roche (Basel, Switzerland). Marigold and paprika oleoresins Soy lecithin (Epikuron 200) was obtained from Lucas Meyer GmbH, Hamburg. The oil was manufactured from minced petals or dried fruits, respectively, by solvent extraction in the country of origin.

Paprika and marigold oleoresins were prepared as follows:

To prepare marigold and paprika oleoresin stock solutions, commercially available marigold and paprika oleoresins (100 mg oils from *Tagetes erecta* L. and *Capsicum annuum* L., respectively) (obtained from Euram Food GmbH (Stuttgart, Germany)) were dissolved in light petroleum ether (25 mL ex BASF, Ludwigshafen, Germany). For determination of the total lutein (marigold) and capsanthin (red paprika) content, the following saponification procedure was performed:

An aliquot (1 ml) of the solution was transferred to a round bottomed flasks, dried in a stream of nitrogen, dissolved in diethyl ether (100 mL ex BASF, Ludwigshafen, Germany), and saponified at room temperature overnight with methanolic potassium hydroxide (30 % w/v, 5 mL ex BASF, Ludwigshafen, Germany). For complete removal of alkali, the solution was washed thrice with 100 ml water each; the organic layer was dried over anhydrous sodium sulphate, filtered through a folded filter, and evaporated to dryness. The residue was dissolved in *tert*-butylmethyl ether/methanol/butylated hydroxytoluene (1:1:0.01 v/v/w, 2 mL ex BASF, Ludwigshafen, Germany and Fluka (Neu-Ulm, Germany) and identified by HPLC analysis.

Aliquots of the oleoresin solutions (1 mL) were transferred into sealable glass tubes (50 mL volume) and the solvent was evaporated in a gentle stream of nitrogen. The enzyme assay was performed in 0.1 M phosphate buffer at pH 7.4 and 37 °C. Bile salts and sodium/calcium ions were used as activators. To improve emulsification, 10 µL of a lecithin solution (250 mg/10 mL methanolen) were added to each tube. For enzymatic hydrolysis, aliquots of a freshly prepared suspension containing lipase in 5 mM calcium chloride solution (12 U/assay) were added and the mixture incubated at 37 °C for 30 min (pre-incubation time before adding the enzyme: 30 min at 37 °C).

The carotenoids were extracted immediately using methanol/ethyl acetate/light petroleum ether (1:1:1, v/v/v, 3 x 20 mL), processed as described above, and identified by HPLC analysis. For each data point, one tube was used (two replicates). The calculation of the conversion rate was based on the areas of the respective capsanthin or lutein peaks, obtained from chemically saponified samples (area = 100 %). Carotenoid monoesters were not taken into account.

Among the 26 commercial lipase preparations tested, with the exception of Chirazyme L-6™ (Roche Diagnostics), a lipase from *Pseudomonas sp.,* only the Candida (*C. rugosa,* formerly known as *C. cylindracea,* and *C. antarctica*) enzymes showed noticeable lipolytic activity towards the lutein and capsanthin esters (table 1) (69% release of capsanthin, 44% of lutein from the respective oleoresins). Likewise, the phospholipases A1 (Lecitase Novo™) and A2 (Lecitase 10L™) did not show any reactivity towards xanthophyll esters. Bile salts were essential for the activity of all commercial enzymes.

The results obtained with the commercial enzymes are summarised in Table 1 below.

**Table 1**

| Genus | Species | Supplier^{a} | Preparation name | Activity | free Capsanthin (paprika) | free Lutein (marigold) |
|---|---|---|---|---|---|---|
| **vertebrates** | | | | | | |
| *Sus* | *scrofa* | 2 | Lecitase 10L | | - ^{b} | - |
| | | | (Phospholip. A2) | | | |
| | *scrofa* | 3 | Pancreatin | 53 U/mg (lip.) | - | - |

| **microorganisms** | | | | | | |
|---|---|---|---|---|---|---|
| *Pseudomo* | *sp.* | 6 | Chirazyme L-6 | 463 U/mg | 44 % | 25 % |
| *nas* | | | | | | |
| *Aspergillus* | *niger* | 3 | Lipase EC | > 120 U/mg | - | - |
| | *oryzae* | 5 | Lipase ex fungal | > 10 U FIP/mg | - | - |
| *Candida* | *rugosa* | 1 | Lipase type Vll | 12 U/mg | 48 % | 18 % |
| | *rugosa* | 3 | Lipase AY | > 30 U/mg | 46 % | 27 % |
| | *rugosa* | 6 | Chirazyme L-3 | 12 U/mg | 30 % | 33 % |
| | *rugosa* | 8 | Gammalip. Z1011 | 30 U/mg | 47 % | 25 % |
| | *cylindracea* | 7 | Sternzym LP 7012 | 10 U/mg | 39 % | 34 % |
| | *cylindracea* | 8 | Gammalip. Z1016 | 360 U/mg | 32 % | 22 % |
| | *antarctica* | 2 | Novozym 868 L | 6 U/mg | 56 % | 44 % |
| | *antarctica (B)* | 2 | Novozym 435 | 10 P-U/mg | - | - |
| | *antarctica* | 6 | Chirazyme L-5 | 72 U/mg | 69 % | 42 % |
| *Fusarium* | *oxysporum* | 2 | Lecitase Novo | - | - | - |
| | | | (Phospholip. A1) | | | |
| *Mucor* | *javanicus* | 3 | Lipase M | > 10 U/mg | - | - |
| *Penicillium* | *camemberti* | 3 | Lipase G "Amano" | 50 U/mg | - | - |
| | *roqueforti* | 3 | Lipase R "Amano" | > 0.9 U/mg | 7 % | - |
| *Rhizomucor* | *miehei* | 2 | Novozym 388 L | 20 U/mg | - | - |
| *Rhizopus* | *oryzae* | 3 | Lipase F-EC | > 150 U/mg | 7 % | - |
| | *oryzae* | 4 | Lilipase A - 10FG | 17 U/mg | - | - |
| | *oryzae* | 8 | GK Lipase | 1 U/mg | - | - |
| | *arrhizus* | 8 | Gammalipase RA | 20-30 U/mg | - | - |
| | *niveus* | 3 | Newlase F | 30 U/mg | - | - |
| | *javanicus* | 7 | Sternzym LP 6012 | 15 U/mg | 5 % | - |
| *Thermomy* | *lanuginosus* | 2 | Lipolase | 100 U/mg | - | - |

| | | | | | | |
|---|---|---|---|---|---|---|
| *ces* ^{a} Suppliers: **1**: Sigma-Aldrich Chemie GmbH, Taufkirchen, Germany; **2**: Novo Nordisk A/S, Bagsvaerd, Denmark; **3**: Extrakt Chemie, Stadthagen, Germany; **4**: Nagase Europe GmbH, Dusseldorf, Germany; **5**: Gustav-G. Diekmann GmbH, Hamburg, Germany; **6**: Roche Diagnostics GmbH, Mannheim, Germany; **7**: Stern-Enzyme GmbH&Co. KG, Ahrensburg, Germany; **8**: Gamma Chemie GmbH, München, Germany. | | | | | | |
| b: "-" stands for conversion < 2 %. | | | | | | |

In Table 1, preparations from genetically modified organisms are named according to the genus and species of the organism from which they were derived. The last two columns designate the amount of free capsanthin and free lutein, liberated in a standard enzymatic assay from paprika oleoresin (*Capsicum annuum* L.) or marigold oleoresin (*Tagetes erecta* L.) and are given in % in relation to the total amount, obtained after chemical saponification of a sample aliquot.

### Example 3

### Bioconversion in submerged cultures of Pleurotus sapidus

The experimental procedures employed were as set out in Example 2. To a submerged culture of *P. sapidus*, growing in a mineral salt medium, the paprika and marigold oleoresins were added separately. Except for the respective oleoresin, no further carbon source or emulsifying agents were present.

After six and eight culture days, respectively, solvent extracts of the mycelia and nutrient media were combined and analysed by means of HPLC with diode array detection. Significant amounts of lutein and capsanthin were liberated from their respective esters by *P. sapidus* submerged cultures. During the transformation period, the pH value shifted moderately from pH 6.65 (day 0) to pH 5.55 (day 6), indicating that enzymatic ester hydrolysis rather than pH correlated hydrolysis had occurred. The desired activity thus was detected in *P. sapidus.* The carotenoid yields after enzymatic saponification and solvent extraction did not sum to 100% (cf. table 2). A co-oxidative carotenoid degradation indirectly catalysed by peroxide producing enzymes may account for the losses of about 10% [Yamauchi R, Miyake N, Inoue H, Kato K Products formed by peroxyl radical oxidation of β-carotene. *J. Agric.* Food *Chem.* 1993, **41**, 708-713].

The experimental procedure was as follows:

All media and equipment were autoclaved prior to use and standard sterile techniques were applied throughout the procedure. A pre-culture of *P. sapidus* (8266 DSM) was grown for seven days in standard nutrient medium (30 gL⁻¹ glucose monohydrate, 3 gL⁻¹ yeast extract, 4.5 gL⁻¹ asparagine monohydrate, 0.5 gL⁻¹ MgSO₄, 1.5 gL⁻¹ KH₂PO₄, 1 mL trace element solution, pH 6.0) at 150 rpm and 24°C. The mycelia were separated by centrifugation (10 min, 3000×g, 4°C) and resuspended into 100 mL of a mineral salt medium (MM) (2 gL⁻¹ NH₄NO₃, 2 gL⁻¹ K₂HPO₄, 1 gL⁻¹ KH₂PO₄, 0.2 gL⁻¹ MgSO₄, 0.14 gL⁻¹ CaCl₂ × 2 H₂O, 0.2 gL⁻¹ NaCl, 2 mL trace element solution, pH 7.0), lacking an additional carbon source. For the main cultures, 100 mL of fresh MM were inoculated with 20 mL of the ultra-turrax homogenised pre-cultures (approximately 100 mg dry mass). 100 mg of paprika or marigold oleoresin were added and the organisms were grown at 150 rpm and 24°C for 6 and 8 days, respectively. The mycelia were separated from the growth media by centrifugation (10 min, 3000×g, 4°C) and both, mycelia and media, were extracted with a mixture of methanol/ethyl acetate/hexane (1:1:1, v/v/v). The organic extracts were combined, concentrated *in vacuo*, made up to volume (10 mL) with *tert*-butyl methyl ether/methanol/butylated hydroxytoluene (1:1:0.01 v/v/w), and identified by HPLC analysis.

The results are shown in Table 2 below.

**Table 2**

| Carotenoid Esters | Conversion of Esters (%) | Number of repetitions (n) | standard deviation |
|---|---|---|---|
| *marigold oleoresin* | | | |
| lutein | 91 | 3 | 4.4 |

| *paprika oleoresin* | | | |
|---|---|---|---|
| cis/trans-capsanthin | 69 | 3 | 3.5 |
| zeaxanthin | 87 | 3 | 5.4 |
| β-cryptoxanthin | 76 | 3 | 3.3 |

Conversion of xanthophyll esters derived from paprika and marigold oleoresins are given in Table 2 in % in relation to the total amount, obtained after laboratory scale chemical saponification of a sample aliquot. As can be seen from Table 2, enzymes obtained from *P. Sapidus* are active in the conversion of carotenoid esters to high levels of conversion, with a high specificity towards lutein and zeaxanthin esters.

### Example 4

### Comparative Example: Bioconversion in submerged cultures of Lentinellus cochleatus

For comparative purposes, the general procedure set out in Example 3 was followed using *L. cochleatus* (L132). No saponification of the xanthophyll esters was observed with the *L. cochleatus* cultures.

### Example 5

### Carotenoid ester hydrolysis with culture supernatant of P. sapidus

Precultures were grown as described by Example 3 above. To induce enzyme secretion into the growth medium, one drop of oleoresin was added to the main cultures. After 6 days, the mycelium was separated by centrifugation and the extracellular enzyme-containing supernatant was employed for the enzymatic conversions. 40 mg of paprika or marigold oleoresin emulsified with 6 g polyethylene glycol tert-octylphenyl ether (Triton-X 100 ™ ex Aldrich) were suspended in 50 mL of main culture supernatant containing 20 mM CaCl₂. The mixture was shaken at 150 rpm and 24°C 14 hours and afterwards extracted and analysed as aforementioned.

By use of mycelium free culture supernatant for the biotransformation, lipase activity was shown to be excreted into the growth medium. As quantification of the conversion yields as well as potential technical downstream processes are substantially simplified in cell free bioprocesses, the following tests were performed with cell free culture supernatant of *P. sapidus.*

To ensure smooth enzyme accessibility, the oleoresins were emulsified with a non-ionic detergent (in this case the commercially available Triton X-100™ ex Aldrich) prior to the addition of the cell free culture medium. Triton X-100™ is considered to be a comparatively mild, non-denaturing detergent that is widely used in biochemical applications.

In contrast to all commercial enzymes tested, no bile salts were needed as activators.

For HPLC separation, a YMC analytical column (YMC Europe, Schermbeck, Germany; 250 x 4.6 mm) with 5 µm-C30-reversed phase material including a precolumn (C30, 10 x 4.0 mm, 5 µm) was used and kept at 35°C. The mobile phase consisted of mixtures of methanol/*tert*-butyl methyl ether/water (81:15:4 v/v/v (A) and 6:90:4 v/v/v (B)), starting with 99% A, followed by a gradient to obtain 56% B after 39 min, 100% B after 45 min, 100% A after 50 min and isocratic 100% A from 50 to 55 min at a flow rate of 1 mL/min. The injection volume was 20 µL. Carotenoids were monitored at 450 nm (DAD; reference: 550 nm, bandwidth: 4 nm each).

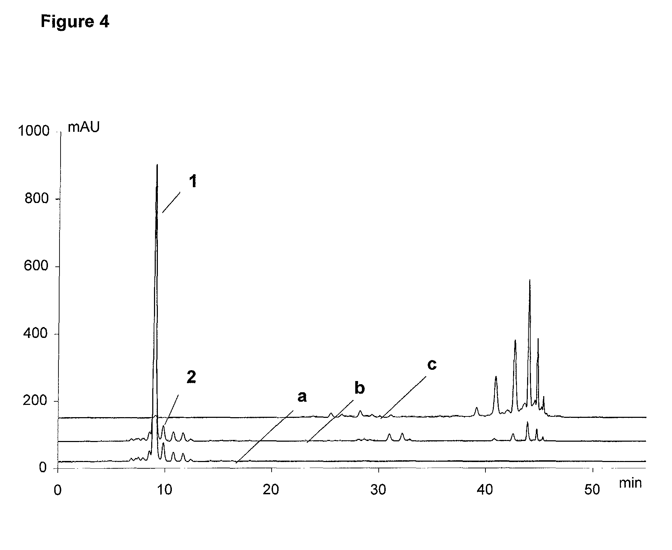

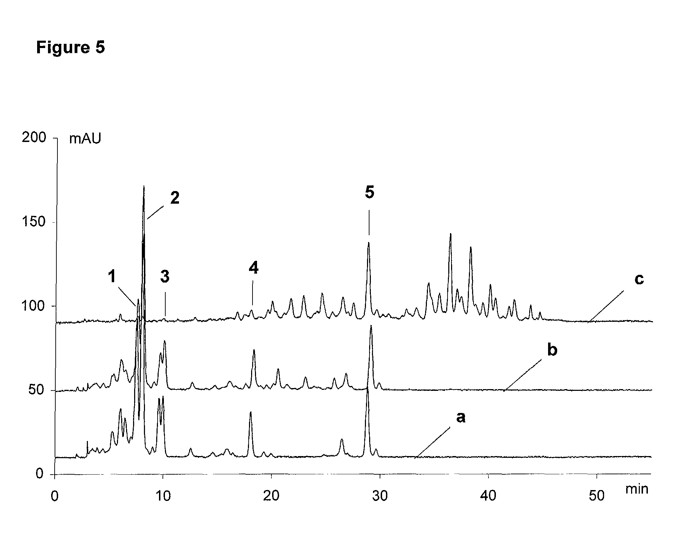

The resulting chromatograms are shown in Figures 4 and 5.

Figure 4 shows a chromatogram (DAD, 450 nm) of samples obtained by chemical saponification of *Tagetes erecta* derived lutein diesters using potassium hydroxide (**a**) or Pleurotus sapidus culture supernatant (**b**). Chromatogram **c** represents the native lutein diester pattern of marigold oleoresin. All chromatograms are in the same scale. Peak assignment: **1** = all-trans-lutein, **2** = zeaxanthin. Peaks between 25 and 35 min correspond to lutein monoesters, and peaks between 38 and 45 min are lutein diesters.

Figure 5 shows a chromatogram (DAD, 450 nm) of samples obtained by chemical saponification of *Capsicum annuum* derived xanthophyll esters (mainly capsanthin diesters) using potassium hydroxide (**a**) or *Pleurotus sapidus* culture supernatant (**b**). Chromatogram c represents the native carotenoid diester pattern of paprika oleoresin. All chromatograms are in the same scale. Peak assignment: **1** = *cis*-capsanthin, **2** = *all-trans*-capsanthin, **3** = zeaxanthin, **4** = β-cryptoxanthin, **5** = *all-trans*-β-carotene. Peaks between 20 and 28 min correspond to monoesters, and peaks between 35 and 45 min are diesters.

Under the experimental conditions applied, nearly complete ester hydrolysis was achieved with both, paprika and marigold oleoresin (figures 4 and 5 and table 2). With parallel analysed heat inactivated culture supernatants no ester cleavage was observed. The progress of liberation of carotenoids from di- via monoesterified to free compounds points to a stepwise saponification process (e.g. figure 4, chromatogram b).

### Example 6

### Examples of detergents used

The following are examples of detergent. ln the detergent compositions, the enzymes levels are expressed by pure enzyme by weight of the total composition and unless otherwise specified, the detergent ingredients are expressed by weight of the total compositions. The abbreviated component identifications therein have the following meanings:

| | |
|---|---|
| LAS | Sodium linear C₁₁₋₁₃ alkyl benzene sulphonate. |
| TAS | Sodium tallow alkyl sulphate. |
| CxyAS | Sodium C₁ₓ - C_{1y} alkyl sulfate. |
| CxySAS | Sodium C₁ₓ - C_{1y} secondary (2,3) alkyl sulfate. |
| MBASx,y | Sodium mid-chain branched alkyl sulfate having an average of x carbon atoms, whereof an average of y carbon atoms are comprised in (a) branching) unit(s) . |
| CxyEz | C₁ₓ - C_{1y} predominantly linear primary alcohol |
| | condensed with an average of z moles of ethylene oxide. |
| CxyEzS | C₁ₓ - C_{1y} sodium alkyl sulfate condensed with an |
| | average of z moles of ethylene oxide. |
| CxEOy | Cy alcohol with an average of ethoxylation of y. |
| Nonionic | Mixed ethoxylated/propoxylated fatty alcohol e.g. Plurafac LF404 being an alcohol with an average degree of ethoxylation of 3.8 and an average degree of propoxylation of 4.5. |
| QAS | R₂.N+(CH₃)₂(C₂H₄OH) with R₂ = C₁₂-C₁₄. |
| SADS | Sodium C14-22 alkyl disulfate of the formula 2-R.C4H7.-1,4-(SO4-)2 where R = C10-18. |
| MES | x-sulpho methyl ester of C18 fatty acid. |
| Soap | Sodium linear alkyl carboxylate derived from a 80/20 mixture of tallow and coconut fatty acids. |
| Silicate | Amorphous Sodium Silicate (SiO₂:Na₂O ratio = 1.6-3.2:1). |
| Metasilicate | Sodium metasilicate (SiO₂:Na₂O ratio = 1.0). |
| Zeolite A | Hydrated Sodium Aluminosilicate of formula Na₁₂(A1O₂SiO₂)₁₂. 27H₂O having a primary particle size in the range from 0.1 to 10 micrometers (Weight expressed on an anhydrous basis). |
| (Na-)SKS-6 | Crystalline layered silicate of formula δ-Na₂Si₂O_{5.} |
| Citrate | Tri-sodium citrate dihydrate. |
| Citric | Anhydrous citric acid. |
| Carbonate | Anhydrous sodium carbonate. |
| Bicarbonate | Sodium hydrogen carbonate. |
| Sulphate | Anhydrous sodium sulphate. |
| STPP | Sodium tripolyphosphate. |
| TSPP | Tetrasodium pyrophosphate. |
| MA/AA | Random copolymer of 4:1 acrylate/maleate, average molecular weight about 70,000-80,000. |
| MA/AA 1 | Random copolymer of 6:4 acrylate/maleate, average molecular weight about 10,000. |
| AA | Sodium polyacrylate polymer of average molecular weight 4,500. |
| Polycarboxylate | Copolymer comprising mixture of carboxylated monomers such as acrylate, maleate and methyacrylate with a MW ranging between 2,000-80,000 such as Sokolan commercially available from BASF, being a copolymer of acrylic acid, MW4,500. |
| BB1 | 3-(3,4-Dihydroisoquinolinium)propane sulfonate as prepared in the preparation example 1 |
| BB2 | 1-(3,4-dihydroisoquinolinium)-decane-2-sulfate as prepared in the preparation example 2 |
| PB1 | Anhydrous sodium perborate monohydrate. |
| PB4 | Sodium perborate tetrahydrate of nominal formula NaBO₃.4H₂O. |
| Percarbonate | Anhydrous sodium percarbonate of nominal formula 2.74 Na₂CO₃.3H₂O₂ . |
| DAP 1 | Diacyl Peroxide Particle with 30% dibenzoyl peroxide, 40% sodium sulfate, 5% Acusol 480N polymer, 2% maltodextrin, 12% ethoxylated stearyl alcohol, and balance as water. |
| DAP 2 | Dilauroyl peroxide available from Akzo |
| NaDCC | Sodium dichloroisocyanurate. |
| TAED | Tetraacetyl ethylene diamine. |
| NOBS | Nonanoyloxybenzene sulfonate in the form of the sodium salt. |
| NACA-OBS | (6-nonamidocaproyl) oxybenzene sulfonate. |
| DOBS | Decanoyl oxybenzene sulfonate in the form of the sodium salt. |
| DTPA | Diethylene triamine pentaacetic acid. |
| HEDP | 1,1-hydroxyethane diphosphonic acid. |
| DETPMP | Diethyltriamine penta (methylene) phosphonate, marketed by Monsanto under the Trade name Dequest 2060. |
| EDDS | Ethylenediamine-N,N'-disuccinic acid, (S,S) isomer in the form of its sodium salt |
| Chelant | Chelant selected from EEDS, HEDP, DTPA, DETPMP and/or mixtures thereof. |
| Catalyst | Mn(bycyclam)Cl2 |
| MnTACN | Manganese 1,4,7-trimethyl-1,4,7-triazacyclononane. |
| Photoactivated Bleach | Sulfonated zinc phtalocyanine encapsulated in dextrin soluble polymer. |
| Photoactivated Bleach 1 | Sulfonated alumino phtalocyanine encapsulated in dextrin soluble polymer. |
| PAAC | Pentaamine acetate cobalt(III) salt. |
| Paraffin | Paraffin oil sold under the tradename Winog 70 by Wintershall. |
| NaBz | Sodium benzoate. |
| Lipase of the present invention | Carotene-specific lipase from *P.sapidus* according to the present invention |
| Protease | Proteolytic enzyme sold under the tradename Savinase , Alcalase, Durazym by Novo Nordisk A/S, Maxacal, Maxapem sold by Gist-Brocades and proteases described in patents WO91/06637 and/or WO95/10591 and/or EP 251 446. |
| Amylase | Amylolytic enzyme sold under the tradename Purafact Ox Am^{R} described in WO 94/18314, WO96/05295 sold by Genencor; Termamyl® , Fungamyl® and Duramyl® , all available from Novo Nordisk A/S and those described in WO95/26397 (sold under the tradename Natalase By Novo Nordisk). |
| Lipase | Lipolytic enzyme sold under the tradename Lipolase Lipolase Ultra by Novo Nordisk A/S and Lipomax by Gist-Brocades. |
| Cellulase | Cellulytic enzyme sold under the tradename Carezyme, Celluzyme and/or Endolase by Novo Nordisk A/S. |
| CMC | Sodium carboxymethyl cellulose. |
| PVNO | Polyvinylpyridine-N-Oxide, with an average molecular weight of 50,000. |
| PVPVI | Copolymer of vinylimidazole and vinylpyrrolidone, with an average molecular weight of 20,000. |
| Brightener 1 | Disodium 4,4'-bis(2-sulphostyryl)biphenyl. |
| Brightener 2 | Disodium 4,4'-bis(4-anilino-6-morpholino-1.3.5-triazin-2-yl) stilbene-2:2'-disulfonate. |
| Silicone antifoam | Polydimethylsiloxane foam controller with siloxaneoxyalkylene copolymer as dispersing agent with a ratio of said foam controller to said dispersing agent of 10:1 to 100:1. |
| Suds Suppressor | 12% Silicone/silica, 18% stearyl alcohol,70% starch in granular form. |
| Thickener | High molecular weight crosslinked polyacrylates such as Carbopol offered by B.F. Goodrich Chemical Company and Polygel. |
| SRP 1 | Anionically end capped poly esters. |
| QEA | bis((C₂H₅O)(C₂H₄O)ₙ)(CH₃) -N⁺-C₆H₁₂-N⁺-(CH₃) bis((C₂H₅O)-(C₂H₄O))ₙ, wherein n = from 20 to 30. |
| PEGX | Polyethylene glycol,of a molecular weight of x. |
| PEO | Polyethylene oxide, with an average molecular weight of 5,000. |
| TEPAE | Tetreaethylenepentaamine ethoxylate. |
| BTA | Benzotriazole. |
| PH | Measured as a 1% solution in distilled water at 20°C. |

### Example 7

The following high density and bleach-containing laundry detergent compositions were prepared according to the present invention:

| | **I** | **II** | **III** | **IV** | **V** | **VI** |
|---|---|---|---|---|---|---|
| Blown Powder | | | | | | |
| Zeolite A | 12.0 | - | 15.0 | 12.0 | - | 15.0 |
| Sulfate | - | 5.0 | - | - | 5.0 | - |
| LAS | 3.0 | - | 3.0 | 3.0 | - | 3.0 |
| C45AS | 3.0 | 2.0 | 4.0 | 3.0 | 2.0 | 4.0 |
| QAS | - | - | 1.5 | - | - | 1.5 |
| DETPMP | 0.4 | 0.4 | 0.4 | 0.4 | 0.4 | 0.4 |
| CMC | 0.4 | 0.4 | 0.4 | 0.4 | 0.4 | 0.4 |
| MA/AA | 1.0 | 2.0 | 2.0 | 1.0 | 2.0 | 2.0 |

| Agglomerates | | | | | | |
|---|---|---|---|---|---|---|
| QAS | 1.0 | - | - | 1.0 | - | - |
| LAS | - | 11.0 | 7.0 | - | 11.0 | 7.0 |
| TAS | 2.0 | 2.0 | 1.0 | 2.0 | 2.0 | 1.0 |
| Silicate | 3.0 | - | 4.0 | 3.0 | - | 4.0 |
| Zeolite A | 8.0 | 8.0 | 8.0 | 8.0 | 8.0 | 8.0 |
| Carbonate Agglomerate | 8.0 | 8.0 | 4.0 | 8.0 | 8.0 | 4.0 |
| NaSKS-6 | 15.0 | 12.0 | 5.0 | 15.0 | 12.0 | 5.0 |
| LAS Spray On | 8.0 | 7.0 | 4.0 | 8.0 | 7.0 | 4.0 |
| Perfume | 0.3 | 0.3 | 0.3 | 0.3 | 0.3 | 0.3 |
| C25E3 | 2.0 | - | 2.0 | 2.0 | - | 2.0 |

| Dry additives | | | | | | |
|---|---|---|---|---|---|---|
| QEA | 1.0 | 0.5 | 0.5 | 1.0 | 0.5 | 0.5 |
| Citric/Citrate | 5.0 | - | 2.0 | 5.0 | - | 2.0 |
| Bicarbonate | - | 3.0 | - | - | 3.0 | - |
| Carbonate | 8.0 | 15.0 | 10.0 | 8.0 | 15.0 | 10.0 |
| TAED and/ or NACA-OBS | 6.0 | - | 5.0 | 6.0 | - | 5.0 |
| NOBS | - | 2.0 | - | - | 2.0 | - |
| DAP 1 | - | - | - | 6.7 | 4.8 | 5.2 |
| Catalyst | 0.002 | - | 0.02 | - | 0.02 | - |
| Percarbonate or PB1 | 14.0 | 7.0 | 10.0 | 4.15 | 7.0 | 10.0 |
| BB1 | 0.40 | - | 0.20 | - | - | - |
| BB2 | - | 0.14 | - | - | - | - |
| Polyethylene oxide of MW 5,000,000 | - | - | 0.2 | - | - | 0.2 |
| Bentonite clay | - | - | 10.0 | - | - | 10.0 |
| Citric acid | 4.0 | - | 1.5 | 4.0 | - | 1.5 |
| Lipase of the present invention | 0.001 | 0.02 | 0.01 | 0.001 | 0.02 | 0.01 |
| Protease | 0.033 | 0.033 | 0.033 | 0.033 | 0.033 | 0.033 |
| Lipase | 0.008 | 0.008 | 0.008 | 0.008 | 0.008 | 0.008 |
| Amylase | 0.001 | 0.001 | 0.001 | 0.001 | 0.001 | 0.001 |
| Cellulase | 0.0014 | 0.0014 | 0.0014 | 0.0014 | 0.0014 | 0.0014 |
| Silicone antifoam | 5.0 | 5.0 | 5.0 | 5.0 | 5.0 | 5.0 |
| Sulfate | - | 3.0 | - | - | 3.0 | - |
| Density (g/litre) | 850 | 850 | 850 | 850 | 850 | 850 |
| Moisture and miscellaneous | Up to 100% | | | | | |

### Example 8

The following laundry compositions, which may be in the form of granules or tablet, were prepared according to the present invention.

| | **I** | **II** | **III** | **IV** | **V** |
|---|---|---|---|---|---|
| Base Product | | | | | |
| C45 AS/TAS | 8.0 | 5.0 | 3.0 | 3.0 | 3.0 |
| LAS | 8.0 | - | 8.0 | - | 7.0 |
| C25AE3S | 0.5 | 2.0 | 1.0 | - | - |
| C25AE5/AE3 | 2.0 | - | 5.0 | 2.0 | 2.0 |
| QAS | - | - | - | 1.0 | 1.0 |
| Zeolite A | 20.0 | 18.0 | 11.0 | - | 10.0 |
| SKS-6 (I) (dry add) | - | - | 9.0 | - | - |
| MA/AA | 2.0 | 2.0 | 2.0 | - | - |
| AA | - | - | - | - | 4.0 |
| Citrate | - | 2.0 | - | - | - |
| Citric | 2.0 | - | 1.5 | 2.0 | - |
| DTPA | 0.2 | 0.2 | - | - | - |
| EDDS | - | - | 0.5 | 0.1 | - |
| HEDP | - | - | 0.2 | 0.1 | - |
| PB1 | 3.0 | 5.0 | 10.0 | - | 4.0 |
| Percarbonate | - | - | - | 18.0 | - |
| NOBS | 3.0 | 4.0 | - | - | 4.0 |
| NACA OBS | - | - | 2.0 | - | - |
| TAED | - | - | 2.0 | 5.0 | - |
| BB1 | 0. 06 | - | 0.34 | - | 0.14 |
| BB2 | - | 0.14 | - | 0.20 | - |
| Catalyst | - | 0.001 | - | - | 0.002 |
| Carbonate | 15.0 | 18.0 | 8.0 | 15.0 | 15.0 |
| Sulphate | 5.0 | 12.0 | 2.0 | 17.0 | 3.0 |
| Silicate | - | 1.0 | - | - | 8.0 |
| Protease | 0.033 | 0.033 | 0.033 | 0.046 | 0.033 |
| Lipase | 0.008 | 0.008 | 0.008 | 0.008 | 0.006 |
| Amylase | 0.001 | 0.001 | 0.001 | 0.0014 | 0.001 |
| Cellulase | 0.0014 | 0.0014 | 0.0014 | 0.01 | - |
| Lipase of the present invention | 0.001 | 0.002 | 0.02 | 0.05 | 0.005 |
| Minors | 0.5 | 0.5 | 0.5 | 0.5 | 0.5 |
| Perfume | 0.2 | 0.3 | 0.5 | 0.2 | 0.1 |
| Moisture and miscellaneous | Up to 100% | | | | |

Minors include Brightener / SRP1 / CMC / Photobleach / MgSO4 / PVPVI/ Suds suppressor /PEG.

### Example 9

The following granular detergent were prepared in accordance with the present invention:

| | **I** | **II** | **III** | **IV** | **V** | **VI** | **VII** | **VIII** |
|---|---|---|---|---|---|---|---|---|
| Base granule | | | | | | | | |
| STPP | - | 22.0 | - | 15.0 | - | 22.0 | - | 15.0 |
| Zeolite A | 30.0 | - | 24.0 | 5.0 | 30.0 | - | 24.0 | 5.0 |
| Sulfate | 5.5 | 5.0 | 7.0 | 7.0 | 5.5 | 5.0 | 7.0 | 7.0 |
| MA/AA | 3.0 | - | - | - | 3.0 | - | - | - |
| AA | - | 1.6 | 2.0 | - | - | 1.6 | 2.0 | - |
| MA/AA (1) | - | 12.0 | - | 6. 0 | - | 12.0 | - | 6.0 |
| LAS | 14.0 | 10.0 | 9.0 | 20.0 | 14.0 | 10.0 | 9.0 | 20.0 |
| C45AS | 8.0 | 7.0 | 9.0 | 7.0 | 8.0 | 7.0 | 9.0 | 7.0 |
| C45AE11 S | - | 1.0 | - | 1.0 | - | 1.0 | - | 1.0 |
| MES | 0.5 | 4.0 | 6.0 | - | 0.5 | 4.0 | 6.0 | - |
| SADS | 2.5 | - | - | 1.0 | 2.5 | - | - | 1.0 |
| Silicate | - | 1.0 | 0.5 | 10.0 | - | 1.0 | 0.5 | 10.0 |
| Soap | - | 2.0 | - | - | - | 2.0 | - | - |
| Brightener 1 | 0.2 | 0.2 | 0.2 | 0.2 | 0.2 | 0.2 | 0.2 | 0.2 |
| Carbonate | 6.0 | 9.0 | 8.0 | 10.0 | 6.0 | 9.0 | 8.0 | 10.0 |
| PEG 4000 | - | 1.0 | 1.5 | - | - | 1.0 | 1.5 | - |
| DTPA | - | 0.4 | - | - | - | 0.4 | - | - |

| Spray on | | | | | | | | |
|---|---|---|---|---|---|---|---|---|
| C25E9 | - | - | - | 5.0 | - | - | - | 5.0 |
| C45E7 | 1.0 | 1.0 | - | - | 1.0 | 1.0 | - | - |
| C23E9 | - | 1.0 | 2.5 | - | - | 1.0 | 2.5 | - |
| Perfume | 0.2 | 0.3 | 0.3 | - | 0.2 | 0.3 | 0.3 | - |

| Dry additives | | | | | | | | |
|---|---|---|---|---|---|---|---|---|
| Carbonate | 5.0 | 10.0 | 13.0 | 8.0 | 5.0 | 10.0 | 13.0 | 8.0 |
| PVPVI/PVNO | 0.5 | - | 0.3 | - | 0.5 | - | 0.3 | - |
| Protease | 0.033 | 0.033 | 0.033 | .0016 | 0.033 | 0.033 | 0.033 | .0016 |
| Lipase | 0.008 | - | - | 0.008 | 0.008 | - | - | 0.008 |
| Amylase | .0016 | - | - | .0016 | .0016 | - | - | .0016 |
| Cellulase | .0002 | .0005 | .0005 | .0002 | .0002 | .0005 | .0005 | .0002 |
| Lipase of the present invention | 0.001 | 0.02 | 0.03 | 0.015 | 0.001 | 0.02 | 0.03 | 0.015 |
| DTPA | 0.5 | 0.3 | 0.5 | 1.0 | 0.5 | 0.3 | 0.5 | 1.0 |
| PB1 | 5 | 3.0 | 10 | 4.0 | 5 | 3.0 | 10 | 4.0 |
| DAP1 | - | - | - | - | 3.8 | 6.7 | 4.3 | 3.2 |
| Catalyst | 0.001 | - | - | 0.002 | - | 0.001 | - | - |
| BB1 | 0.2 | - | - | 0.5 | - | - | - | - |
| BB2 | - | 0.3 | 0.4 | - | - | - | - | - |
| NOBS/ TAED | 0.5 | 0.3 | 0.5 | 0.6 | 0.5 | 0.3 | 0.5 | 0.6 |
| Sulfate | 4.0 | 5.0 | - | 5.0 | 4.0 | 5.0 | - | 5.0 |
| SRP1 | - | 0.4 | - | - | - | 0.4 | - | - |
| Sud supressor | - | 0.5 | - | - | - | 0.5 | - | - |
| speckle | 0.9 | - | 2.7 | 1.2 | 0.9 | - | 2.7 | 1.2 |
| Moisture and miscellaneous | Up to 100% | | | | | | | |

### Example 10

The following laundry detergent compositions were prepared in accordance with the present invention:

| | **I** | **II** | **III** | **IV** | **V** | **VI** | **VII** | **VIII** |
|---|---|---|---|---|---|---|---|---|
| LAS | 13.3 | 13.7 | 10.4 | 8.0 | 13.3 | 13.7 | 10.4 | 8.0 |
| C₄₅ AS | 3.9 | 4.0 | 4.5 | - | 3.9 | 4.0 | 4.5 | - |
| C₄₅ E0.5S | 2.0 | 2.0 | - | - | 2.0 | 2.0 | - | - |
| C45 E3S | - | - | - | - | - | - | - | - |
| C45E6.5S | 0.5 | 0.5 | 0.5 | 5.0 | 0.5 | 0.5 | 0.5 | 5.0 |
| C₉-C₁₄ alkyl dimethyl hydroxy ethyl quaternary NH4 salt | 1.0 | - | - | 0.5 | 1.0 | - | - | 0.5 |
| Tallow fatty acid | 0.5 | - | - | - | 0.5 | - | - | - |
| Tallow alcohol ethoxylate (50) | - | - | 1.0 | 0.3 | - | - | 1.0 | 0.3 |
| STPP | - | 41.0 | - | 20.0 | - | 41.0 | - | 20.0 |
| Zeolite A | 26.3 | - | 21.3 | 1.0 | 26.3 | - | 21.3 | 1.0 |
| Carbonate | 23.9 | 12.4 | 25.2 | 17.0 | 23.9 | 12.4 | 25.2 | 17.0 |
| Sodium Polyacrylate (45%) | 3.4 | 0.0 | 2.7 | - | 3.4 | 0.0 | 2.7 | - |
| MA/AA | - | - | 1.0 | 1.5 | - | - | 1.0 | 1.5 |
| Silicate (1:6 ratio) | 2.4 | 6.4 | 2.1 | 6.0 | 2.4 | 6.4 | 2.1 | 6.0 |
| Sulfate | 10.5 | 10.9 | 8.2 | 15.0 | 10.5 | 10.9 | 8.2 | 15.0 |
| PB1 | 1.0 | 1.0 | 1.0 | 2.0 | 1.0 | 1.0 | 1.0 | 2.0 |
| PEG MW 4000 (50%) | 1.7 | 0.4 | 1.0 | - | 1.7 | 0.4 | 1.0 | - |
| CMC | 1.0 | - | - | 0.3 | 1.0 | - | - | 0.3 |
| Citric | - | - | 3.0 | - | - | - | 3.0 | - |
| BB1 | 1.0 | 0.5 | 0.6 | - | - | - | - | - |
| BB2 | - | 0.2 | - | 1.0 | - | - | - | - |
| DAP 1 | - | - | - | - | 2.0 | 2.1 | 3.4 | 2.1 |
| NOBS/ DOBS | 0.2 | 0.5 | 0.5 | 0.1 | - | - | - | - |
| TAED | 0.6 | 0.5 | 0.4 | 0.3 | - | - | - | - |
| SRP 1 | 1.5 | - | - | - | 1.5 | - | - | - |
| SRP2 | - | 1.5 | 1.0 | 1.0 | - | 1.5 | 1.0 | 1.0 |
| Moisture | 7.5 | 3.1 | 6.1 | 7.3 | 7.5 | 3.1 | 6.1 | 7.3 |
| Mn sulphate | - | - | - | 1.0 | - | - | - | 1.0 |
| Chelant | - | - | - | 0.5 | - | - | - | 0.5 |
| speckles | 0.5 | 1.0 | 3.0 | 0.5 | 0.5 | 1.0 | 3.0 | 0.5 |
| Protease | 0.033 | 0.033 | 0.033 | 0.046 | 0.033 | 0.033 | 0.033 | 0.046 |
| Lipase | 0.008 | 0.008 | 0.008 | 0.008 | 0.008 | 0.008 | 0.008 | 0.008 |
| Amylase | 0.001 | 0.001 | 0.001 | .0014 | 0.001 | 0.001 | 0.001 | .0014 |
| Cellulase | .0014 | .0014 | .0014 | 0.01 | .0014 | .0014 | .0014 | 0.01 |
| Lipase of the present invention | 0.001 | 0.01 | 0.005 | 0.002 | 0.001 | 0.01 | 0.005 | 0.002 |
| Minors | 1.0 | 1.0 | 1.0 | 1.0 | 1.0 | 1.0 | 1.0 | 1.0 |

### Example 11

The following granular fabric detergent compositions which provide "softening through the wash" capability were prepared according to the present invention :

| | **I** | **II** | **III** | **IV** |
|---|---|---|---|---|
| C45AS | - | 10.0 | - | 10.0 |
| LAS | 7.6 | - | 7.6 | - |
| C68AS | 1.3 | - | 1.3 | - |
| C45E7 | 4.0 | - | 4.0 | - |
| C25E3 | - | 5.0 | - | 5.0 |
| Coco-alkyl-dimethyl hydroxyethyl ammonium chloride | 1.4 | 1.0 | 1.4 | 1.0 |
| Citrate | 5.0 | 3.0 | 5.0 | 3.0 |
| Na-SKS-6 | - | 11.0 | - | 11.0 |
| Zeolite A | 15.0 | 15.0 | 15.0 | 15.0 |
| MA/AA | 4.0 | 4.0 | 4.0 | 4.0 |
| DETPMP | 0.4 | 0.4 | 0.4 | 0.4 |
| DAP 1 | 4.8 | 6.7 | - | - |
| Catalyst | - | - | 0.001 | 0.001 |
| Percarbonate | - | - | - | 15.0 |
| PB1 | - | - | 15.0 | - |
| TAED | - | - | 5.0 | 5.0 |
| Smectite clay | 10.0 | 10.0 | 10.0 | 10.0 |
| HMWPEO | - | 0.1 | - | 0.1 |
| Lipase of the present invention | 0.001 | 0.01 | 0.001 | 0.01 |
| Protease | 0.02 | 0.01 | 0.02 | 0.01 |
| Lipase | 0.02 | 0.01 | 0.02 | 0.01 |
| Amylase | 0.03 | 0.005 | 0.03 | 0.005 |
| Cellulase | 0.001 | - | 0.001 | - |
| Silicate | 3.0 | 5.0 | 3.0 | 5.0 |
| Carbonate | 10.0 | 10.0 | 10.0 | 10.0 |
| Suds suppressor | 1.0 | 4.0 | 1.0 | 4.0 |
| CMC | 0.2 | 0.1 | 0.2 | 0.1 |
| Miscellaneous and minors | Up to 100% | | | |

### Example 12

The following liquid detergent formulations were prepared according to the present invention (Levels are given in parts per weight, enzyme are expressed in pure enzyme) :

| | **I** | **II** | **III** | **IV** | **V** |
|---|---|---|---|---|---|
| LAS | 11.5 | 9.0 | - | 4.0 | - |
| C25E2.5S | - | 3.0 | 18.0 | - | 16.0 |
| C45E2.25S | 11.5 | 3.0 | - | 16.0 | - |
| C23E9 | - | 3.0 | 2.0 | 2.0 | 1.0 |
| C23E7 | 3.2 | - | - | - | - |
| CFAA | - | - | 5.0 | - | 3. 0 |
| TPKFA | 2.0 | - | 2.0 | 0.5 | 2.0 |
| Citric (50%) | 6.5 | 1.0 | 2.5 | 4.0 | 2.5 |
| Ca formate | 0.1 | 0.06 | 0.1 | - | - |
| Na formate | 0.5 | 0.06 | 0.1 | 0.05 | 0.05 |
| SCS | 4.0 | 1.0 | 3.0 | 1.2 | - |
| Borate | 0.6 | - | 3.0 | 2.0 | 3.0 |
| Na hydroxide | 6.0 | 2.0 | 3.5 | 4.0 | 3.0 |
| Ethanol | 2.0 | 1.0 | 4.0 | 4.0 | 3.0 |
| 1,2 Propanediol | 3.0 | 2.0 | 8.0 | 8.0 | 5.0 |
| Monoethanolamine | 3.0 | 1.5 | 1.0 | 2.5 | 1.0 |
| TEPAE | 2.0 | - | 1.0 | 1.0 | 1.0 |
| Catalyst | 0.01 | 0.01 | 0.005 | 0.005 | 0.1 |
| Lipase of the present invention | 0.001 | 0.002 | 0.01 | 0.01 | 0.005 |
| Protease | 0.03 | 0.01 | 0.03 | 0.02 | 0.02 |
| Lipase | - | - | 0.002 | - | - |
| Amylase | - | - | - | 0.002 | - |
| Cellulase | - | - | 0.0002 | 0.0005 | 0.0001 |
| SRP 1 | 0.2 | - | 0.1 | - | - |
| DTPA | - | - | 0.3 | - | - |
| PVNO | - | - | 0.3 | - | 0.2 |
| Brightener 1 | 0.2 | 0.07 | 0.1 | - | - |
| Silicone antifoam Miscellaneous and water | 0.04 | 0.02 | 0.1 | 0.1 | 0.1 |

### Example 13

The following laundry bar detergent compositions were prepared according to the present invention (Levels are given in parts per weight, enzyme are expressed in pure enzyme) :

| | **I** | **II** | **III** | **VI** | **V** | **III** | **VI** | **V** |
|---|---|---|---|---|---|---|---|---|
| LAS | - | - | 19.0 | 15.0 | 21.0 | 6.75 | 8.8 | - |
| C28AS | 30.0 | 13.5 | - | - | - | 15.75 | 11.2 | 22.5 |
| Na Laurate | 2.5 | 9.0 | - | - | - | - | - | - |
| Zeolite A | 2.0 | 1.25 | - | - | - | 1.25 | 1.25 | 1.25 |
| Carbonate | 20.0 | 3.0 | 13.0 | 8.0 | 10.0 | 15.0 | 15.0 | 10.0 |
| Ca Carbonate | 27.5 | 39.0 | 35.0 | - | - | 40.0 | - | 40.0 |
| Sulfate | 5.0 | 5.0 | 3.0 | 5.0 | 3.0 | - | - | 5.0 |
| TSPP | 5.0 | - | - | - | - | 5.0 | 2.5 | - |
| STPP | 5.0 | 15.0 | 10.0 | - | - | 7.0 | 8.0 | 10.0 |
| Bentonite clay | - | 10.0 | - | - | 5.0 | - | - | - |
| DETPMP | - | 0.7 | 0.6 | - | 0.6 | 0.7 | 0.7 | 0.7 |
| CMC | - | 1.0 | 1.0 | 1.0 | 1.0 | - | - | 1.0 |
| Talc | - | - | 10.0 | 15.0 | 10.0 | - | - | - |
| Silicate | - | - | 4.0 | 5.0 | 3.0 | - | - | - |
| PVNO | 0.02 | 0.03 | - | 0.01 | - | 0.02 | - | - |
| MA/AA | 0.4 | 1.0 | - | - | 0.2 | 0.4 | 0.5 | 0.4 |
| SRP 1 | 0.3 | 0.3 | 0.3 | 0.3 | 0.3 | 0.3 | 0.3 | 0.3 |
| Lipase of the present invention | 0.01 | 0.001 | 0.005 | 0.02 | 0.02 | 0.001 | 0.01 | 0.01 |
| Amylase | - | - | 0.01 | - | - | - | 0.002 | - |
| Protease | - | 0.004 | - | 0.003 | 0.003 | - | - | 0.003 |
| Lipase | - | 0.002 | - | 0.002 | - | - | - | - |
| Cellulase | - | .0003 | - | - | .0003 | .0002 | - | - |
| Catalyst | 1.0 | 5.0 | 0.1 | 3.0 | 10.0 | 1.0 | 0.3 | 1.0 |
| PEO | - | 0.2 | - | 0.2 | 0.3 | - | - | 0.3 |
| Perfume | 1.0 | 0.5 | 0.3 | 0.2 | 0.4 | - | - | 0.4 |
| Mg sulfate | - | - | 3.0 | 3.0 | 3.0 | - | - | - |
| Brightener | 0.15 | 0.1 | 0.15 | - | - | - | - | 0.1 |
| Photoactivated bleach (ppm) | - | 15.0 | 15.0 | 15.0 | 15.0 | - | - | 15.0 |

### Example 14

The following laundry bar detergent compositions were prepared according to the present invention (Levels are given in parts per weight, enzyme are expressed in pure enzyme) :

| | **I** | **II** | **III** | **IV** | **V** | **VI** | **VII** | **VIII** |
|---|---|---|---|---|---|---|---|---|
| LAS | - | - | 19.0 | 15.0 | 21.0 | 6.75 | 8.8 | - |
| C28AS | 30.0 | 13.5 | - | - | - | 15.75 | 11.2 | 22.5 |
| Na Laurate | 2.5 | 9.0 | - | - | - | - | - | - |
| Zeolite A | 2.0 | 1.25 | - | - | - | 1.25 | 1.25 | 1.25 |
| Carbonate | 8.0 | 3.0 | 1.0 | 8.0 | 10.0 | 15.0 | 3.0 | 10.0 |
| Ca Carbonate | 27.5 | 27.0 | 35.0 | - | - | 28.0 | - | 28.0 |
| Sulfate | 5.0 | 5.0 | 3.0 | 5.0 | 3.0 | - | - | 5.0 |
| TSPP | 5.0 | - | - | - | - | 5.0 | 2.5 | - |
| STPP | 5.0 | 15.0 | 10.0 | - | - | 7.0 | 8.0 | 10.0 |
| Bentonite clay | - | 10.0 | - | - | 5.0 | - | - | - |
| DETPMP | - | 0.7 | 0.6 | - | 0.6 | 0.7 | 0.7 | 0.7 |
| CMC | - | 1.0 | 1.0 | 1.0 | 1.0 | - | - | 1.0 |
| Talc | - | - | 10.0 | 15.0 | 10.0 | - | - | - |
| Silicate | - | - | 4.0 | 5.0 | 3.0 | - | - | - |
| PVNO | 0.02 | 0.03 | - | 0.01 | - | 0.02 | - | - |
| MA/AA | 0.4 | 1.0 | - | - | 0.2 | 0.4 | 0.5 | 0.4 |
| SRP 1 | 0.3 | 0.3 | 0.3 | 0.3 | 0.3 | 0.3 | 0.3 | 0.3 |
| Lipase of the present invention | 0.01 | 0.001 | 0.005 | 0.02 | 0.02 | 0.1 | 0.01 | 0.01 |
| Amylase | - | - | 0.01 | - | - | - | 0.002 | - |
| Protease | - | 0.004 | - | 0.003 | 0.003 - | | - | 0.003 |
| Lipase | - | 0.002 | - | 0.002 | - | - | - | - |
| Cellulase | - | .0003 | - | - | .0003 | .0002 | - | - |
| PEO | - | 0.2 | - | 0.2 | 0.3 | - | - | 0.3 |
| Perfume | 1.0 | 0.5 | 0.3 | 0.2 | 0.4 | - | - | 0.4 |
| Mg sulfate | - | - | 3.0 | 3.0 | 3.0 | - | - | - |
| Brightener | 0.15 | 0.1 | 0.15 | - | - | - | - | 0.1 |
| Catalyst | 0.001 | - | - | 0.001 | - | - | - | - |
| BB1 | 0.2 | 0.2 | 0.3 | - | - | 0.4 | - | - |
| BB2 | - | - | - | 0.4 | 0.5 | - | 0.45 | 0.3 |
| TAED | 1.7 | 1.7 | 1.7 | 1.7 | 1.7 | 1.7 | 1.7 | 1.7 |
| PB4 | 12.0 | 12.0 | 12.0 | 12.0 | 12.0 | 12.0 | 12.0 | 12.0 |
| NOBS | 0.2 | 0.2 | 0.2 | 0.20 | 0.2 | 0.2 | 0.2 | 0.2 |
| Photoactivated bleach (ppm) | - | 15.0 | 15.0 | 15.0 | 15.0 | - | - | 15.0 |

### Example 15

The following compact high density (0.96Kg/l) dishwashing detergent compositions were prepared according to the present invention :

| | **I** | **II** | **III** | **lV** | **V** | **VI** |
|---|---|---|---|---|---|---|
| STPP | - | 51.0 | 51.0 | - | - | 44.3 |
| Citrate | 17.0 | - | - | 50.0 | 40.2 | - |
| Carbonate | 17.5 | 14.0 | 20.0 | - | 8.0 | 33.6 |
| Bicarbonate | - | - | - | 26.0 | - | - |
| Silicate | 15.0 | 15.0 | 8.0 | - | 25.0 | 3.6 |
| Metasilicate | 2.5 | 4.5 | 4.5 | - | - | - |
| PB1 | 10.0 | 8.0 | 8.0 | - | - | - |
| PB4 | - | - | - | 10.0 | - | - |
| Percarbonate | - | - | - | - | 11.8 | 4.8 |
| BB1 | - | 0.1 | 0.1 | - | 0.5 | - |
| BB2 | 0.2 | 0.05 | - | 0.1 | - | 0.6 |
| Nonionic | 2.0 | 1.5 | 1.5 | 3.0 | 1.9 | 5.9 |
| TAED | 2.0 | - | - | 4.0 | - | 1.4 |
| HEDP | 1.0 | - | - | - | - | - |
| DETPMP | 0.6 | - | - | - | - | - |
| MnTACN | - | - | - | - | 0.01 | - |
| PAAC | - | 0.01 | 0.01 | - | - | - |
| Paraffin | 0.5 | 0.4 | 0.4 | 0.6 | - | - |
| Lipase of the present invention | 0.04 | 0.1 | 0.03 | 0.5 | 0.005 | 0.005 |
| Protease | 0.072 | 0.053 | 0.053 | 0.026 | 0.059 | 0.01 |
| Amylase | 0.012 | 0.012 | 0.012 | 0.021 | 0.021 | 0.006 |
| Lipase | - | 0.001 | - | 0.005 | - | - |
| BTA | 0.3 | 0.2 | 0.2 | 0.3 | 0.3 | 0.3 |
| Polycarboxylate | 6.0 | - | - | - | 4.0 | 0.9 |
| Perfume | 0.2 | 0.1 | 0.1 | 0.2 | 0.2 | 0.2 |
| pH | 11.0 | 11.0 | 11.3 | 9.6 | 10.8 | 10.9 |
| Miscellaneous, sulfate and water | Up to 100% | | | | | |

### Example 16

The following tablet detergent compositions were prepared according to the present invention by compression of a granular dishwashing detergent composition at a pressure of 13KN/cm² using a standard 12 head rotary press:

| | **I** | **II** | **III** | **IV** | **V** | **VI** | **VII** | **VIII** |
|---|---|---|---|---|---|---|---|---|
| STPP | - | 48.8 | 54.7 | 38.2 | - | 52.4 | 56.1 | 36.0 |
| Citrate | 20.0 | - | - | - | 35.9 | - | - | - |
| Carbonate | 20.0 | 5.0 | 14.0 | 15.4 | 8.0 | 23.0 | 20.0 | 28.0 |
| Silicate | 15.0 | 14.8 | 15.0 | 12.6 | 23.4 | 2.9 | 4.3 | 4.2 |
| Lipase of the present invention | 0.001 | 0.001 | 0.01 | 0.004 | 0.02 | 0.02 | 0.001 | 0.005 |
| Protease | 0.042 | 0.072 | 0.042 | 0.031 | 0.052 | 0.023 | 0.023 | 0.029 |
| Amylase | 0.012 | 0.012 | 0.012 | 0.007 | 0.015 | 0.003 | 0.017 | 0.002 |
| Lipase | 0.005 | - | - | - | - | - | - | - |
| PB1 | 14.3 | 7.8 | 11.7 | 12.2 | - | - | 6.7 | 8.5 |
| PB4 | - | - | - | - | 22.8 | - | 3.4 | - |
| Percarbonate | - | - | - | - | - | 10.4 | - | - |
| BB1 | 0.2 | - | 0.5 | - | 0.3 | 0.2 | - | - |
| BB2 | - | 0.2 | - | 0.5 | - | - | 0.1 | 0.2 |
| Nonionic | 1.5 | 2.0 | 2.0 | 2.2 | 1.0 | 4.2 | 4.0 | 6.5 |
| PAAC | - | - | 0.02 | 0.009 | - | - | - | - |
| MnTACN | - | - | - | - | 0.007 | - | - | - |
| TAED | 2.7 | 2.4 | - | - | - | 2.1 | 0.7 | 1.6 |
| HEDP | 1.0 | - | - | 0.9 | - | 0.4 | 0.2 | - |
| DETPMP | 0.7 | - | - | - | - | - | - | - |
| Paraffin | 0.4 | 0.5 | 0.5 | 0.5 | - | - | 0.5 | - |
| BTA | 0.2 | 0.3 | 0.3 | 0.3 | 0.3 | 0.3 | 0.3 | - |
| Polycarboxylate | 4.0 | - | - | - | 4.9 | 0.6 | 0.8 | - |
| PEG | - | - | - | - | - | 2.0 | - | 2.0 |
| Glycerol | - | - | - | - | - | 0.4 | - | 0.5 |
| Perfume | - | - | - | 0.05 | 0.2 | 0.2 | 0.2 | 0.2 |
| Weight of tablet | 20g | 25g | 20g | 30g | 18g | 20g | 25g | 24g |
| pH | 10.7 | 10.6 | 10.7 | 10.7 | 10.9 | 11.2 | 11.0 | 10.8 |
| Miscellaneous, sulfate and water | Up to 100% | | | | | | | |

### Example 17

The following compact high density (0.96Kg/1) dishwashing detergent compositions were prepared according to the present invention :

| | **I** | **II** | **III** | **IV** | **V** | **VI** |
|---|---|---|---|---|---|---|
| STPP | - | 51.0 | 51.0 | - | - | 44.3 |
| Citrate | 17.0 | - | - | 50.0 | 40.2 | - |
| Carbonate | 17.5 | 14.0 | 20.0 | - | 8.0 | 33.6 |
| Bicarbonate | - | - | - | 26.0 | - | - |
| Silicate | 15.0 | 15.0 | 8.0 | - | 25.0 | 3.6 |
| Metasilicate | 2.5 | 4.5 | 4.5 | - | - | - |
| PB1 | 10.0 | 8.0 | 8.0 | - | - | - |
| PB4 | - | - | - | 10.0 | - | - |
| Percarbonate | - | - | - | - | 11.8 | 4.8 |
| Nonionic | 2.0 | 1.5 | 1.5 | 3.0 | 1.9 | 5.9 |
| DAP 1 | 0.2 | 1.0 | 4.3 | 6.7 | 1.7 | 0.3 |
| TAED | 2.0 | - | - | 4.0 | - | 1.4 |
| HEDP | 1.0 | - | - | - | - | - |
| DETPMP | 0.6 | - | - | - | - | - |
| MnTACN | - | - | - | - | 0.01 | - |
| PAAC | - | 0.01 | 0.01 | - | - | - |
| Paraffin | 0.5 | 0.4 | 0.4 | 0.6 | - | - |
| Lipase of the present invention | 0.04 | 0.001 | 0.03 | 0.005 | 0.005 | 0.005 |
| Protease | 0.072 | 0.053 | 0.053 | 0.026 | 0.059 | 0.01 |
| Amylase | 0.012 | 0.012 | 0.012 | 0.021 | 0.021 | 0.006 |
| Lipase | - | 0.001 | - | 0.005 | - | - |
| BTA | 0.3 | 0.2 | 0.2 | 0.3 | 0.3 | 0.3 |
| Polycarboxylate | 6.0 | - | - | - | 4.0 | 0.9 |
| Perfume | 0.2 | 0.1 | 0.1 | 0.2 | 0.2 | 0.2 |
| PH | 11.0 | 11.0 | 11.3 | 9.6 | 10.8 | 10.9 |
| Miscellaneous, sulfate and water | Up to 100% | | | | | |

### Example 18

The following tablet detergent compositions were prepared according to the present invention by compression of a granular dishwashing detergent composition at a pressure of 13KN/cm² using a standard 12 head rotary press:

| | **I** | **II** | **III** | **IV** | **V** | **VI** | **VII** | **VIII** |
|---|---|---|---|---|---|---|---|---|
| STPP | - | 48.8 | 54.7 | 38.2 | - | 52.4 | 56.1 | 36.0 |
| Citrate | 20.0 | - | - | - | 35.9 | - | - | - |
| Carbonate | 20.0 | 5.0 | 14.0 | 15.4 | 8.0 | 23.0 | 20.0 | 28.0 |
| Silicate | 15.0 | 14.8 | 15.0 | 12.6 | 23.4 | 2.9 | 4.3 | 4.2 |
| Lipase of the present invention | 0.1 | 0.001 | 0.01 | 0.4 | 0.02 | 0.02 | 0.1 | 0.005 |
| Protease | 0.042 | 0.072 | 0.042 | 0.031 | 0.052 | 0.023 | 0.023 | 0.029 |
| Amylase | 0.012 | 0.012 | 0.012 | 0.007 | 0.015 | 0.003 | 0.017 | 0.002 |
| Lipase | 0.005 | - | - | - | - | - | - | - |
| PB1 | 14.3 | 7.8 | 11.7 | 12.2 | - | - | 6.7 | 8.5 |
| PB4 | - | - | - | - | 22.8 | - | 3.4 | - |
| Percarbonate | - | - | - | - | - | 10.4 | - | - |
| DAP 1 | 0.6 | 0.8 | 1.0 | 1.2 | 1.1 | 0.8 | 0.5 | 1.4 |
| Nonionic | 1.5 | 2.0 | 2.0 | 2.2 | 1.0 | 4.2 | 4.0 | 6.5 |
| PAAC | - | - | 0.02 | 0.009 | - | - | - | - |
| MnTACN | - | - | - | - | 0.007 | - | - | - |
| TAED | 2.7 | 2.4 | - | - | - | 2.1 | 0.7 | 1.6 |
| HEDP | 1.0 | - | - | 0.9 | - | 0.4 | 0.2 | - |
| DETPMP | 0.7 | - | - | - | - | - | - | - |
| Paraffin | 0.4 | 0.5 | 0.5 | 0.5 | - | - | 0.5 | - |
| BTA | 0.2 | 0.3 | 0.3 | 0.3 | 0.3 | 0.3 | 0.3 | - |
| Polycarboxylate | 4.0 | - | - | - | 4.9 | 0.6 | 0.8 | - |
| PEG | - | - | - | - | - | 2.0 | - | 2.0 |
| Glycerol | - | - | - | - | - | 0.4 | - | 0.5 |
| Perfume | - | - | - | 0.05 | 0.2 | 0.2 | 0.2 | 0.2 |
| Weight of tablet | 20g | 25g | 20g | 30g | 18g | 20g | 25g | 24g |
| PH | 10.7 | 10.6 | 10.7 | 10.7 | 10.9 | 11.2 | 11.0 | 10.8 |
| Miscellaneous, sulfate and water | Up to 100% | | | | | | | |

### Example 19

The following compact high density (0.96Kg/l) dishwashing detergent compositions were prepared according to the present invention :

| | **I** | **II** | **III** | **IV** | **V** | **VI** |
|---|---|---|---|---|---|---|
| STPP | - | 51.0 | 51.0 | - | - | 44.3 |
| Citrate | 17.0 | - | - | 50.0 | 40.2 | - |
| Carbonate | 17.5 | 14.0 | 20.0 | - | 8.0 | 33.6 |
| Bicarbonate | - | - | - | 26.0 | - | - |
| Silicate | 15.0 | 15.0 | 8.0 | - | 25.0 | 3.6 |
| Metasilicate | 2.5 | 4.5 | 4.5 | - | - | - |
| Catalyst | 0.01 | 0.005 | 0.1 | 2.0 | 0.01 | 0.005 |
| PB1 | 10.0 | 8.0 | 8.0 | - | - | - |
| PB4 | - | - | - | 10.0 | - | - |
| Percarbonate | - | - | - | - | 11.8 | 4.8 |
| Nonionic | 2.0 | 1.5 | 1.5 | 3.0 | 1.9 | 5.9 |
| TAED | 2.0 | - | - | 4.0 | - | 1.4 |
| HEDP | 1.0 | - | - | - | - | - |
| DETPMP | 0.6 | - | - | - | - | - |
| PAAC | - | 0.01 | 0.01 | - | - | - |
| Paraffin | 0.5 | 0.4 | 0.4 | 0.6 | - | - |
| Lipase of the present invention | 0.04 | 0.001 | 0.03 | 0.005 | 0.005 | 0.005 |
| Protease | 0.072 | 0.053 | 0.053 | 0.026 | 0.059 | 0.01 |
| Amylase | 0.012 | 0.012 | 0.012 | 0.021 | 0.021 | 0.006 |
| Lipase | - | 0.001 | - | 0.005 | - | - |
| BTA | 0.3 | 0.2 | 0.2 | 0.3 | 0.3 | 0.3 |
| Polycarboxylate | 6.0 | - | - | - | 4.0 | 0.9 |
| Perfume | 0.2 | 0.1 | 0.1 | 0.2 | 0.2 | 0.2 |
| PH | 11.0 | 11.0 | 11.3 | 9.6 | 10.8 | 10.9 |
| Miscellaneous, sulfate and water | Up to 100% | | | | | |

### Example 20

The following tablet detergent compositions were prepared according to the present invention by compression of a granular dishwashing detergent composition at a pressure of 13KN/cm² using a standard 12 head rotary press:

| | **I** | **II** | **III** | **IV** | **V** | **VI** | **VII** | **VIII** |
|---|---|---|---|---|---|---|---|---|
| STPP | - | 48.8 | 54.7 | 38.2 | - | 52.4 | 56.1 | 36.0 |
| Citrate | 20.0 | - | - | - | 35.9 | - | - | - |
| Carbonate | 20.0 | 5.0 | 14.0 | 15.4 | 8.0 | 23.0 | 20.0 | 28.0 |
| Silicate | 15.0 | 14.8 | 15.0 | 12.6 | 23.4 | 2.9 | 4.3 | 4.2 |
| Lipase of the present invention | 0.001 | 0.001 | 0.01 | 0.004 | 0.02 | 0.02 | 0.001 | 0.005 |
| Protease | 0.042 | 0.072 | 0.042 | 0.031 | 0.052 | 0.023 | 0.023 | 0.029 |
| Amylase | 0.012 | 0.012 | 0.012 | 0.007 | 0.015 | 0.003 | 0.017 | 0.002 |
| Lipase | 0.005 | - | - | - | - | - | - | - |
| Catalyst | 0.001 | 0.003 | 0.05 | 0.001 | 0.001 | 0.003 | 0.01 | 0.001 |
| PB1 | 14.3 | 7.8 | 11.7 | 12.2 | - | - | 6.7 | 8.5 |
| PB4 | - | - | - | - | 22.8 | - | 3.4 | - |
| Percarbonate | - | - | - | - | - | 10.4 | - | - |
| Nonionic | 1.5 | 2.0 | 2.0 | 2.2 | 1.0 | 4.2 | 4.0 | 6.5 |
| PAAC | - | - | 0.02 | 0.009 | - | - | - | - |
| TAED | 2.7 | 2.4 | - | - | - | 2.1 | 0.7 | 1.6 |
| HEDP | 1.0 | - | - | 0.9 | - | 0.4 | 0.2 | - |
| DETPMP | 0.7 | - | - | - | - | - | - | - |
| Paraffin | 0.4 | 0.5 | 0.5 | 0.5 | - | - | 0.5 | - |
| BTA | 0.2 | 0.3 | 0.3 | 0.3 | 0.3 | 0.3 | 0.3 | - |
| Polycarboxylate | 4.0 | - | - | - | 4.9 | 0.6 | 0.8 | - |
| PEG | - | - | - | - | - | 2.0 | - | 2.0 |
| Glycerol | - | - | - | - | - | 0.4 | - | 0.5 |
| Perfume | - | - | - | 0.05 | 0.2 | 0.2 | 0.2 | 0.2 |
| Weight of tablet | 20g | 25g | 20g | 30g | 18g | 20g | 25g | 24g |
| PH | 10.7 | 10.6 | 10.7 | 10.7 | 10.9 | 11.2 | 11.0 | 10.8 |
| Miscellaneous, sulfate and water | Up to 100% | | | | | | | |

### Example 21

The following liquid rinse aid compositions were prepared according to the present invention :

| | **I** | **II** | **III** | **IV** |
|---|---|---|---|---|
| Lipase of the present invention | 0.001 | 0.0005 | 0.01 | 0.001 |
| Catalyst | 0.1 | 0.01 | 0.008 | 0.001 |
| Nonionic | 10.0 | 13.6 | 62.3 | 60.0 |
| Propylene glycol | - | - | 5.0 | 5.5 |
| Citric | 3.5 | 4.6 | - | - |
| SCS | 10.0 | 7.7 | - | - |
| pH of the liquid | 3.0 | 2.5 | 7.2 | 7.2 |
| Miscellaneous, solvent and water | Up to 100% | | | |

### Example 22

The following automatic dishwashing tablets were made in accordance with the present invention (g of raw material and enzymes are expressed in pure enzyme):

| | **I** | **II** | **III** | **IV** | **V** | **VI** |
|---|---|---|---|---|---|---|
| Phase 1 | | | | | | |
| STPP | 9.6 | 9.6 | 10.6 | 9.6 | 9.6 | 10.6 |
| Silicate | 0.5 | 0.7 | 2.9 | 0.5 | 0.7 | 2.9 |
| SKS-6 | 1.5 | 1.5 | - | 1.5 | 1.5 | - |
| Carbonate | 2.3 | 2.7 | 2.8 | 2.3 | 2.7 | 2.8 |
| HEDP | 0.2 | 0.2 | 0.2 | 0.2 | 0.2 | 0.2 |
| PB1 | 2.4 | 2.4 | 2.8 | 2.4 | 2.4 | 2.8 |
| PAAC | 0.002 | 0.002 | - | - | - | - |
| Catalyst | - | - | - | 0.002 | 0.002 | - |
| BB1 | 0.2 | 0.5 | - | - | - | - |
| DAP 1 | - | - | 0.5 | - | - | 0.2 |
| Amylase | 0.1 | 0.1 | 0.001 | 0.1 | 0.1 | 0.001 |
| Protease | 0.06 | 0.06 | 0.002 | 0.06 | 0.06 | 0.002 |
| Nonionic | 0.4 | 0.8 | 0.4 | 0.4 | 0.8 | 0.4 |
| PEG 6000 | 0.4 | 0.26 | - | 0.4 | 0.26 | - |
| BTA | 0.04 | 0.04 | 0.06 | 0.04 | 0.04 | 0.06 |
| Paraffin | 0.1 | 0.10 | 0.1 | 0.1 | 0.10 | 0.1 |
| Perfume | 0.02 | 0.02 | 0.02 | 0.02 | 0.02 | 0.02 |
| Total | 17.7g | 18.5g | 20.1g | 17.7g | 18.5g | 20.1 g |

| Phase 2 | | | | | | |
|---|---|---|---|---|---|---|
| Lipase of the present invention | 0.005 | 0.5 | 0.2 | 0.005 | 0.5 | 0.2 |
| Amylase | 0.003 | 0.003 | 0.004 | 0.003 | 0.003 | 0.004 |
| Protease | 0.01 | 0.009 | 0.01 | 0.01 | 0.009 | 0.01 |
| Citric acid | 0.3 | - | 0.6 | 0.3 | - | 0.6 |
| Sulphamic acid | - | 0.3 | - | - | 0.3 | - |
| Bicarbonate | 1.1 | 0.4 | 0.6 | 1.1 | 0.4 | 0.6 |
| Carbonate | - | 0.5 | - | - | 0.5 | - |
| Triacetin | - | - | 1.2 | - | - | 1.2 |
| CaCl₂ | - | 0.07 | 0.1 | - | 0.07 | 0.1 |
| PEG 6000 | - | - | 1.2 | - | - | 1.2 |
| PEG 3000 | 0.06 | 0.06 | - | 0.06 | 0.06 | - |
| Total | 2.05g | 2.50g | 23.6g | 2.05g | 2.50g | 23.6g |

The tablet compositions and II are prepared as follows. The detergent active composition of phase 1 is prepared by admixing the granular and liquid components and is then passed into the die of a conventional rotary press. The press includes a punch suitably shaped for forming a mould. The cross-section of the die is approximately 30x38 mm. The composition is then subjected to a compression force of 940 kg/cm² and the punch is then elevated exposing the first phase of the tablet containing the mould in its upper surface. The detergent active composition of phase 2 is prepared in similar manner and is passed into the die. The particulate active composition is then subjected to a compression force of 170 kg/cm², the punch is elevated, and the multi-phase tablet ejected from the tablet press. The resulting tablets dissolve or disintegrate in a washing machine as described above within 12 minutes, phase 2 of the tablets dissolving within 5 minutes. The tablets display improved strength, especially on long-term storage, together with excellent dissolution characteristics.

The tablet composition III was prepared as follows : The compressed portion is prepared by delivering the composition of active detergent components to a punch cavity of a modified rotary tablet press and compressing the composition at a pressure of 940kg/cm². The modified tablet press provides tablet wherein the compressed portion has a mould. For the purposes of Example lll, the non-compressed portion is in particulate form. The non-compressed portion is accurately delivered to the mould of the compressed portion using a nozzle feeder. The non-compressed portion is adhered to the compressed portion by coating the non-compressed portion with a coating layer which contacts the compressed portion.

## Claims

1. A lipase enzyme derived from *Pleurotus sapidus* capable of catalysing the enzymatic hydrolysis of carotenoid esters to release free carotenoids.

2. The lipase enzyme according to claim 1, wherein it has an isoelectric point of about 5.7 and a molecular weight of about 101 kDa.

3. The lipase enzyme according to claim 1 or 2, wherein it is active in the absence of bile salts.

4. The lipase enzyme according to claim 1, 2 or 3, wherein it has a substrate specificity for xanthophyll esters.

5. The lipase enzyme according to claim 4, wherein the xanthophyll esters are selected from capsanthin ester, *cis*-capsanthin ester, *all-trans*-capsanthin ester, zeaxanthin ester, and lutein ester.

6. The lipase enzyme according to any one of the preceding claims, wherein it displays a conversion efficiency of at least 69%, preferably at least 80%, more preferably at least 90%.

7. A method for producing free carotenoids from their ester derivatives, comprising contacting a carotenoid ester with the lipase enzyme according to any one of claims 1 to 6.

8. The method according to claim 7, further comprising isolating free carotenoids.

9. The method of claim 7 or 8, wherein the carotenoid ester is contacted with the lipase enzyme in an environment substantially free of bile salts.

10. The method according to any on of claims 7 to 9, wherein a cell-free culture supernatant of *Pleurotus sapidus* containing the lipase enzyme according to any on of claims 1 to 6 is employed.

11. The method according to any one of claims 7 to 10, wherein the carotenoid ester compound is an oleoresin derived from a plant source, in particular *Capsicum annuum* L. and/or *Tagetes erecta* L.

12. The method according to any one of claims 7 to 11, wherein the carotenoid ester compound is a xanthophyll ester.

13. The method according to claim 12, wherein the xanthophyll ester is selected from capsanthin ester, cis-capsanthin ester, *all-trans*-capsanthin ester, zeaxanthin ester, lutein ester, diesterified β-cryptoxanthin, capsombin, mutatoxanthin, luteoxanthin and violaxanthin.

14. The method according to any one of claims 7 to 13, wherein the free carotenoids are suitable for use as precursors of fragrances and/or flavours in perfumes and/or foods and as colorants.

15. A method for treating carotene-comprising stains, comprising contacting the stain with a lipase enzyme according to any one of claims 1 to 6.

16. The method according to claim 15, further comprising contacting the stain with a detergent and rinsing to at least partially remove the stain.

17. The method according to claim 15 or 16, wherein the stain is additionally contacted with an enzyme which cleaves carotenoids.

18. A detergent composition, comprising a lipase enzyme according to any one of claims 1 to 6.

19. The detergent composition according to claim 18, further comprising an enzyme capable of cleaving free carotenoids.

20. The detergent composition according to claim 18 or 19, further comprising a surfactant, dispersant, balance carrier and/or adjunct ingredient.

21. Use of the lipase enzyme according to any one of claims 1 to 6 or a cell-free culture supernatant of *Pleurotus sapidus* for the production of free carotenoids from their respective carotenoid esters.

22. Use of the lipase enzyme according to any one of claims 1 to 6 or a cell-free culture supernatant of *Pleurotus sapidus* for the treatment of stains.
